# EUROPEAN PATENT SPECIFICATION

(11) **EP 1 587 926 B1**
(45) Date of publication and mention of the grant of the patent: **18.07.2007**
(21) Application number: 04704041.5
(22) Date of filing: 21.01.2004
(51) Int. Cl.: C12N 15/11, A61K 31/713, A61P 31/14

(54) **LIPOPHILIC DERIVATIVES OF DOUBLE-STRANDED RIBONUCLEIC ACID**
LIPOPHILE DERIVATE DOPPELSTRÄNGIGER RIBONUKLEINSÄURE
DERIVES LIPOPHILES D'ACIDE RIBONUCLEIQUE A DOUBLE BRIN

(30) Priority: 21.01.2003 DE 10302421
(43) Date of publication of application: 26.10.2005
(60) Divisional application: 07010839.4
(73) Proprietor: Alnylam Europe AG, 95326 Kulmbach (DE)
(72) Inventor: HADWIGER, Philipp, 96264 Altenkunstadt (DE); JOHN, Matthias, 96103 Hallstadt (DE); LORENZ, Christina, 1160 Wien (DE); VORNLOCHER, Hans-Peter, 95448 Bayreuth (DE); LIMMER, Stefan, 95326 Kulmbach (DE)
(74) Representative: Ehnis, Tobias
(86) International application number: PCT/US2004/001461
(87) International publication number: WO 2004/065601

(56) References cited:
- WO-A-02/055693
- TUSCHL ET AL: "Small interfering RNAs: a revolutionary tool for the analysis of gene function and gene therapy" MOLECULAR INTERVENTIONS, vol. 2, 2002, pages 158-167, XP002294002
- MANOHARAN: "Oligonucleotide conjugates as potential antisense drugs with improved uptake, biodistribution, targeted delivery, and mechanism of action" ANTISENSE NUCLEIC ACID DRUG DEVELOPMENT, vol. 12, 2002, pages 103-128, XP002294027 cited in the application
- CHIU ET AL: "RNAi in human cells: Basic structural and functional features of small interfering RNA" MOLECULAR CELL, vol. 10, 2002, pages 549-561, XP002978510 cited in the application
- SCHWARZ ET AL: "Evidence that siRNAs function as guides, not primers, in the Drosophila and human RNAi patways" MOLECULAR CELL, vol. 10, 2002, pages 537-548, XP002257820 cited in the application

## Description

### Field of the Invention

This invention relates to double-stranded ribonucleic acid (dsRNA) having improved RNA interference activity, and its use in mediating RNA interference in vitro and in vivo.

### Background of the Invention

Many diseases (e.g., cancers, hematopoietic disorders, endocrine disorders, and immune disorders) arise from the abnormal expression or activity of a particular gene or group of genes. Similarly, disease can result through expression of a mutant form of protein, as well as from expression of viral genes that have been integrated into the genome of their host. The therapeutic benefits of being able to selectively silence these abnormal or foreign genes is obvious.

Several therapeutic agents capable of inhibiting the expression of a target gene have been developed, most notably antisense nucleic acid (see, e.g., Skorski, T. et al., Proc. Natl. Acad. Sci. USA (1994) 91:4504-4508). However, antisense approaches, which use either single-stranded RNA or DNA, act in a 1:1 stoichiometric relationship and thus have low efficacy (Skorski et al., *supra*). For example, Jansen et al. report that relatively high doses (1.7 mg/kg body weight per day, resulting in long term plasma concentrations above 1 mg/l) of antisense RNA specific for the gene bcl2 were required to attain the intended effect of the antisense compound (i.e., 40 % reduction in bcl2 expression) (Jansen, B., et al., The Lancet (2000) 356:1728-1733).

Considerable research efforts have focused on mechanisms for improving the efficiency of inhibition by antisense oligonucleotides, which must be transported across cell membranes or taken up by cells in order to reach their target and become biologically active. One method for increasing membrane or cellular transport is by the attachment of a lipophilic group. Manoharan, M., Antisense Nucl. Acid Drug Dev. (2002) 12:103-128, and U.S Patent Application Publ. No. 20030064492 (Manoharan) disclose the conjugation of antisense oligonucleotides to various functional groups, including steroids and non-aromatic lipophilic molecules, to assist in the transfer of the antisense agent across cellular membranes. Ramirez, et al., J. Am. Chem. Soc. (1982) 104:5483, discloses the introduction of the phospholipid group 5'-O-(1,2-di-O-myristoyl-sn-glycero-3-phosphoryl) into the dimer TpT independently at the 3' and 5' positions. Shea, et al., Nucl. Acids Res. (1990) 18:-3777, discloses oligonucleotides having a 1,2-di-O-hexyldecyl-rac-glycerol group linked to a 5'-phosphate on the 5'-terminus of the oligonucleotide. Bijsterbosch, M.K., et al., Nucl. Acids Res. (2000) 28(14):2717-2725, discloses conjugation of phosphorothioate antisense oligodeoxynucleotides with cholesterol to increase interactions with receptors and plasma proteins, thereby enhancing cellular uptake of the antisense oligonucleotides.

Relatively recently, double-stranded RNA molecules (dsRNA) have been shown to block gene expression in a highly conserved regulatory mechanism known as RNA interference (RNAi). Briefly, the RNAse III Dicer enzyme processes dsRNA into small interfering RNAs (siRNA) of approximately 22 nucleotides, one strand of which (the "guide strand") then serves as a guide sequence to induce cleavage of messenger RNA (mRNA) by an RNA-induced silencing complex (RISC) of mRNAs comprising a nucleotide sequence which is complementary to the sequence of the guide strand (Hammond, S.M., et al., Nature (2000) 404:293-296). In other words, RNAi involves a catalytic-type reaction whereby new siRNAs are generated through successive cleavage of long dsRNA. Thus, unlike antisense, RNAi degrades target RNA in a non-stoichiometric manner. When administered to a cell or organism, exogenous dsRNA designed to comprise a sequence on one of its strands (the "complementary strand") which is complementary to a sequence in an endogenous target mRNA molecule has been shown to direct the sequence-specific degradation of the target mRNA through RNAi. For example, Kreutzer, R., Limmer, S., International PCT Publication No.WO 00/44895 discloses dsRNA that are effective agents for inducing RNAi, as well as methods for introducing dsRNA into a cell to inhibit the expression of a target gene.

It has been observed that blocking the hydroxyl group located at the 5'-terminus of the complementary strand in an siRNA in a manner that precludes its phosphorylation by nucleic acid kinases abolishes the ability of the siRNA to interfere with the expression of its target gene in *Drosophila* embryo lysates (Nykänen et al., Cell (2001) 107:309-321). Schwarz, D.S., et al. (Mol. Cell (2002) 10:537-548) employed end-modified siRNA to confirm the conservation of the RNAi mechanism between flies and mammals. After blocking the 5'-OH of the complementary strand by methylation, the endogenous kinase which usually phosphorylates the 5'-OH of dsRNA, was not able to add a 5'-phosphate to the siRNA and consequently RNAi activity was largely abolished., both in Drosophila extracts as well as in cultured human HeLa cells. However, a 5'-phosphodiester on the antisense strand, i.e. 6-aminohexyl phosphodiester, rendered the resulting duplex active in target mRNA silencing, although activity was reduced compared to a 5'-OH bearing duplex.

Chiu, Y.L., and Rana, T.M., (Mol. Cell (2002) 10:549-561) investigated the contribution of the 3'- and 5'-termini to dsRNA activity by employing dsRNA with end-modified sense or antisense strands in reporter-cotransfection experiments in HeLa cells. Whereas 5'-termini of both strands where conjugated to an aminopropyl phosphodiester, Puromycin, and Biotin were attached to both dsRNA 3'-ends. Modification of the 3'-ends was well tolerated, irrespectively whether the sense or the antisense was altered. This picture changed for the 5'-modification, where the modifying entity was tolerated exclusively on the sense strand. In contradiction to the results of Schwarz, D.S., et al. (Mol. Cell (2002) 10:537-548), this 5'-phosphodiester-modification of the antisense strand abolished RNAi activity. Czauderna, F., et al. (Nucleic Acids Research (2003) 31:2705-2716) similarly found reduced gene expression inhibitory activity in dsRNA modified at the 5'-end of the complementary strand.

From WO 02/055693 a dsRNA is known that exhibits a double-stranded structure made up from a maximum of 49 successive nucleotide pairs, and wherein one strand or at least one segment of that one strand of the double-stranded structure is complementary to a target gene.

From Tuschl, T. and Borkhardt, A., Molecular Interventions, Vol. 2, 2002, pages 158-167 a dsRNA with attached lipophilic residues is known. The lipophilic residues are attached at the 3'-termini of the siRNA duplex.

Despite efforts in increasing the efficiency of antisense technology, particularly by enhancing uptake of antisense oligonucleotides by cells, there is currently no known means for improving the efficiency of RNA interference by dsRNA. Thus, there remains a need for a more effective dsRNA molecule that can selectively and efficiently silence a target gene. More specifically, a dsRNA molecule that has high biological activity and can be readily synthesized would be highly desirable. Compositions comprising such agents would be useful for treating diseases caused by abnormal expression or activity of a gene.

### Summary of the Invention

The present invention discloses double-stranded ribonucleic acid (dsRNA) having improved RNA interference activity, methods of making the dsRNA, as well as compositions and methods for inhibiting the expression of a target gene in a cell using the dsRNA- The present invention also discloses compositions for treating diseases caused by the expression or activity of the target gene. The dsRNA of the invention comprises an RNA strand (complementary RNA strand) having a region which is complementary to an RNA transcript of at least a part of a target gene, and one covalently linked lipophilic group.

The invention relates to double-stranded ribonucleic acid (dsRNA) having improved biological activity. The dsRNA comprises a complementary RNA strand having a nucleotide sequence that is complementary to at least a part of a target gene and one covalently linked lipophilic group. The lipophilic group may be an aromatic, aliphatic or alicyclic moiety, or any combination thereof. The lipophilic group may be a steroid, such as a sterol (e.g., cholesterol), or an aliphatic hydrocarbon, such as a branched aliphatic hydrocarbon, or a combination thereof. In a particular embodiment, the lipophilic group is cholesteryl (6-hydroxyhexyl) carbamate or 12-hydroxydodecanoic acid bisdecylamide. The dsRNA further comprises a second (sense) RNA strand. The lipophilic group is covalently linked to the 5'-terminus of the complementary RNA strand wherein the covalent linkage comprises a phosphodiester bond. In another embodiment, the lipophilic group is linked to the 5'-terminus of the sense strand, and the covalent linkage may comprise a phosphodiester bond. The complementary RNA strand or the sense RNA strand may comprise a nucleotide overhang of 1 to 4, preferably 1 or 2, nucleotides in length. The nucleotide overhang may be on the 3'-terminus of the complementary RNA strand, and the 5'-end of the RNA strand may be blunt. The complementary RNA strand is between 16 and 30 nucleotides in length, preferably between 16 and 25 nucleotides in length, and most preferably between 20 and 25 nucleotides in length. The second (sense) RNA strand may have the same number (or fewer) nucleotides as the complementary RNA strand. The octanol-water partition coefficient, logK_{ow}, of the lipophilic group is greater than 1, preferably greater than 1.5, 2, 3, 4 or 5.

The present invention encompasses a double stranded RNA molecule comprising a complementary RNA strand and one lipophilic group having a log K_{ow} exceeding 1, wherein the complementary RNA strand has a nucleotide sequence which is complementary to a target RNA and where the target RNA is an mRNA transcript of a portion of a target gene or a portion of a (+) strand RNA virus, wherein the lipophilic group is covalently linked to the 5' end of the sense strand. In one embodiment, the lipophilic group is covalently linked to the 5' end of the sense strand by a covalent linkage which does not comprise a phosphodiester group.

In another aspect, the invention relates to a method for inhibiting the expression of a target gene in a cell. The method comprises introducing a dsRNA, as described above, into the cell, and maintaining the cell for a time sufficient to obtain degradation of a mRNA transcript of the target gene. The cell may be a hepatocyte, a pancreatic cell, a uterine cell, or a cell of the cervix or urinary bladder. The liver cell may be an endothelial cell, a Kupffer cell or a parenchymal cell. The target gene may be a viral gene, for example a (+) strand RNA virus such as a Hepatitis C Virus (HCV). In a particular embodiment, the target gene is at least a portion of a 3'-untranslated region (3'-UTR) of a (+) strand RNA virus, such as a 3'-UTR of HCV.

In yet another aspect, the invention relates to a pharmaceutical composition for inhibiting the expression of a target gene in an organism. The compositions comprises a dsRNA, as described above, and a pharmaceutically acceptable carrier. The cell organism may be a mammal, such as a human. The dosage unit of dsRNA may be less than 5 milligram (mg) of dsRNA per kg body weight of the mammal, in a range of 0.01 to 2.5 milligrams (mg), 0.1 to 200 micrograms (µg), 0.1 to 100 µg per kilogram body weight of the mammal, or less than 25 µg per kilogram body weight of the mammal. The pharmaceutically acceptable carrier may be an aqueous solution, such as phosphate buffered saline, or it may comprise a micellar structure, such as a liposome, capsid, capsoid, polymeric nanocapsule, or polymeric microcapsule.

The details of once or more embodiments of the invention are set forth in the accompanying drawings and the description below. Other features, objects, and advantages of the invention will be apparent from the description and drawings, and from the claims.

### Brief Description of the Figures

FIG. 1 is a diagram of a synthetic scheme for the cholesterol derivative "Chol."
FIG. 2 is a diagram of a synthetic scheme for the di-n-decylamine derivative "C32."
FIG. 3 shows the relative β**-**galactosidase activity in β-Gal+HuH-7 cells following transfection with dsRNA.
FIG. 4 shows the relative β-galactosidase activity in β-Gal+HuH-7 cells following transfection with dsRNA without use of a transfection aid, and a Northern blot analysis of the dsRNA isolated from the cells after the transfection.
FIG. 5 shows a Northern blot analysis of dsRNA isolated from cells of the (a) pancreas, (b) uterus, and (c) urinary bladder following transfection without the use of a transfection aid.

### Detailed Description of the Invention

The present invention discloses double-stranded ribonucleic acid (dsRNA) having improved biological activity, methods of making the dsRNA, as well as compositions and methods for inhibiting the expression of a target gene in a cell using the dsRNA. The present invention also discloses compositions for treating diseases in organisms caused by expression of a target gene using dsRNA. dsRNA directs the sequence-specific degradation of mRNA through a process known as RNA interference (RNAi). The process occurs in a wide variety of organisms, including mammals and other vertebrates. The dsRNA of the invention comprises an RNA strand (complementary RNA strand) having a region that is complementary to an RNA transcript of at least a portion of a target gene, and one pendant lipophilic group having a log K_{ow} exceeding 1 covalently attached to the 5'-end of the complementary RNA strand or the sense RNA strand. The lipophilic group may be an aromatic, an aliphatic or an alicyclic moiety, or any combination thereof. For example, the lipophilic group may be a sterol, such as cholesterol or an aliphatic hydrocarbon, such as a branched aliphatic hydrocarbon (e.g., cholesteryl (6-hydroxyhexyl) carbamate or 12-hydroxydodecanoic acid bisdecylamide). Using a cell-based assay, the present inventors have demonstrated that these conjugated lipophilic dsRNA can specifically and efficiently mediate RNAi, regardless of the mechanism of cellular uptake, resulting in significant inhibition of expression of the target gene. The present invention encompasses these dsRNAs and compositions comprising dsRNA and their use for specifically inactivating gene function. The use of these dsRNA enables the targeted degradation of mRNAs of genes that are implicated in a wide variety of disease processes, including those implicated in (+)RNA strand viral infections, such as Hepatitis C. Thus, the methods and compositions of the present invention comprising these dsRNA are useful for treating diseases and disorders caused by the expression or activity of a particular gene, such as HCV and HCV-associated diseases.

The following detailed description discloses how to make and use the dsRNA and compositions containing dsRNA to inhibit the expression of a target gene, as well as compositions for treating diseases and disorders caused by the expression of the gene. The pharmaceutical compositions of the present invention comprise a dsRNA having a complementary nucleotide sequence of between 16 and 30 nucleotides in length, preferably between 16 and 25 nucleotides in length, more preferably between 20 and 25 nucleotides in length, and most preferably between 21 and 24 nucleotides in length, and which is substantially identical to at least a part of the target gene (e.g., a 3'-UTR of a (+) strand RNA virus), together with a pharmaceutically acceptable carrier.

Accordingly, certain aspects of the present invention relate to pharmaceutical compositions comprising the dsRNA of the present invention together with a pharmaceutically acceptable carrier.

### 1. Definitions

For convenience, the meaning of certain terms and phrases used in the specification, examples, and appended claims, are provided below.

As used herein, "target gene" refers to a section of a DNA strand of a double-stranded DNA that is complementary to a section of a DNA strand, including all transcribed regions, that serves as a matrix for transcription, as well as a section of an RNA strand of a (+) strand RNA virus. A target gene, usually the sense strand, is a gene whose expression is to be selectively inhibited or silenced through RNA interference. The term "target gene" specifically encompasses any cellular gene or gene fragment whose expression or activity is associated with a disease or disorder (e.g., an oncogene), as well as any foreign or exogenous gene or gene fragment whose expression or activity is associated with a disease, such as a gene from a pathogenic organism (e.g., a viral or pro-viral gene, viroid, or plasmodium). The target gene may be a viral gene, for example a (+) strand RNA virus such as a Hepatitis C Virus (HCV). In a particular embodiment, the target gene is at least a portion of a 3'-untsanslated region (3'-UTR) of a (+) strand RNA virus, such as a 3'-UTR of HCV.

Examples of genes which can be targeted for treatment include, without limitation, an oncogene (Hanahan, D. and R.A. Weinberg, Cell (2000) 100:57; and Yokota, J., Carcinogenesis (2000) 21(3):497-503); a cytokine gene (Rubinstein, M., et al., Cytokine Growth Factor Rev. (1998) 9(2):175-81); a idiotype (Id) protein gene (Benezra, R., et al., Oncogene (2001) 20(58):8334-41; Norton, J.D., J. Cell Sci. (2000) 113(22):3897-905); a prion gene (Prusiner, S.B., et al., Cell (1998) 93(3):337-48; Safar, J., and S.B. Prusiner, Prog. Brain Res. (1998) 117:421-34); a gene that expresses molecules that induce angiogenesis (Gould, V.E. and B.M. Wagner, Hum. Pathol. (2002) 33(11):1061-3); adhesion molecules (Chothia, C. and E.Y. Jones, Annu. Rev. Biochem. (1997) 66:823-62; Parise, L.V., et al., Semin. Cancer Biol. (2000) 10(6):407-14); cell surface receptors (Deller, M.C., and Y.E. Jones, Curr. Opin. Struct. Biol. (2000) 10(2):213-9); genes of proteins that are involved in metastasizing and/or invasive processes (Boyd, D., Cancer Metastasis Rev. (1996) 15(1):77-89; Yokota, J., Carcinogenesis (2000) 21(3):497-503); genes of proteases as well as of molecules that regulate apoptosis and the cell cycle (Matrisian, L.M., Curr. Biol. (1999) 9(20):R776-8; Krepela, E., Neoplasma (2001) 48(5):332-49; Basbaum and Werb, Curr. Opin. Cell Biol. (1996) 8:731-738; Birkedal-Hansen, et al., Crit. Rev. Oral Biol. Med. (1993) 4:197-250; Mignatti and Rifkin, Physiol. Rev. (1993) 73:161-195; Stetler-Stevenson, et al., Annu. Rev. Cell Biol. (1993) 9:541-573; Brinkerhoff, E., and L.M. Matrisan, Nature Reviews (2002) 3:207-214; Strasser, A., et al., Annu. Rev. Biochem. (2000) 69:217-45; Chao, D.T. and S.J. Korsmeyer, Annu. Rev. Immunol. (1998) 16:395-419; Mullauer, L., et al., Mutat. Res. (2001) 488(3):211-31; Fotedar, R., et al., Prog. Cell Cycle Res. (1996) 2:147-63; Reed, J.C., Am. J. Pathol. (2000) 157(5):1415-30; D'Ari, R., Bioassays (2001) 23(7):563-5); genes that express the EGF receptor; Mendelsohn, J. and J. Baselga, Oncogene (2000) 19(56):6550-65; Normanno, N., et al., Front. Biosci. (2001) 6:D685-707); and the multidrug resistance 1 gene, MDR1 gene (Childs, S., and V. Ling, Imp. Adv. Oncol. (1994) 21-36).

In a preferred embodiment, the target gene is a gene that is expressed in hepatocytes, pancreatic cells, and cells of the reproductive or urinary systems (e.g., the uterus, cervix, or urinary bladder). The hepatocyte may be an endothelial cell, a Kupffer cell or a parenchymal cell. The dsRNA of the present invention are particularly active in cells of the reproductive and urinary systems, such as those listed above, and thus are particularly useful for treating infectious diseases of these organs, such as the Hepatitis C virus and HCV-like viruses that target these cells.

As used herein, the terms "3'-untranslated region" and "3'-UTR" refer to the conserved, non-coding region at the 3'-end of a viral genome. The 3'-UTR can be the entire non-coding region or a fragment thereof. As used herein, the term "highly conserved region" refers to a region of the viral genome that remains evolutionarily constant, i.e., a genomic region that has a very low mutation rate and thus shares significant sequence identity (>99%) between distinct viral genotypes.

The term "complementary RNA strand" (also referred to herein as the "antisense strand") refers to the strand of a dsRNA which is complementary to an mRNA transcript that is formed during expression of the target gene, or its processing products, or a region (such as the 3'-UTR) of a (+) strand RNA virus. As used herein, the term "complementary nucleotide sequence" refers to the region on the complementary RNA strand that is complementary to a region of an mRNA transcript of the target gene or a region (e.g., 3'-UTR) of a (+) strand RNA virus. "dsRNA" refers to a ribonucleic acid molecule having a duplex structure comprising two complementary and anti-parallel nucleic acid strands. Not all nucleotides of a dsRNA must exhibit Watson-Crick base pairs; the two RNA strands may be substantially complementary (i.e., having no more than one or two nucleotide mismatches). The maximum number of base pairs is the number of nucleotides in the shortest strand of the dsRNA where the dsRNA forms a duplex structure. The RNA strands may have the same or a different number of nucleotides. The complementary RNA strand has between 16 and 30, preferably between 16 and 25, more preferably between 20 and 25 nucleotides in length, and most preferably between 21 and 24 nucleotides in length.

"Introducing into" means uptake or absorption in the cell, as is understood by those skilled in the art. Absorption or uptake of dsRNA can occur through cellular processes, or by auxiliary agents or devices, such as viral and synthetic vectors. For example, for in vivo delivery, dsRNA can be injected into a tissue site or administered systemically. In vitro delivery includes methods known in the art such as electroporation and lipofection.

As used herein, a "nucleotide overhang" refers to the unpaired nucleotide or nucleotides that protrude from the duplex structure when a 3'-end of one RNA strand extends beyond the 5'-end of the other complementary strand, or vice versa. "Blunt" or "blunt end" means that the lengths of the two RNA strand are the same at that end of the dsRNA, and hence there is no nucleotide(s) protrusion (i.e., no nucleotide overhang).

As used herein and as known in the art, the term "identity" is the relationship between two or more polynucleotide sequences, as determined by comparing the sequences. Identity also means the degree of sequence relatedness between polynucleotide sequences, as determined by the match between strings of such sequences. Identity can be readily calculated (see, e.g., Computation Molecular Biology, Lesk, A.M., eds., Oxford University Press, New York (1998), and Biocomputing: Informatics and Genome Projects, Smith, D.W., ed., Academic Press, New York (1993), both of which are incorporated by reference herein). While there exist a number of methods to measure identity between two polynucleotide sequences, the term is well known to skilled artisans (see, e.g., Sequence Analysis in Molecular Biology, von Heinje, G., Academic Press (1987); and Sequence Analysis Primer, Gribskov., M. and Devereux, J., eds., M. Stockton Press, New York (1991)). Methods commonly employed to determine identity between sequences include, for example, those disclosed in Carillo, H., and Lipman, D., SIAM J. Applied Math. (1988) 48:1073. "Substantially identical," as used herein, means there is a very high degree of homology (preferably 100% sequence identity) between the sense strand of the dsRNA and the corresponding part of the target 3'-UTR of the viral genome. However, dsRNA having greater than 90%, or 95% sequence identity may be used in the present invention, and thus sequence variations that might be expected due to genetic mutation, strain polymorphism, or evolutionary divergence can be tolerated. Although 100% identity is preferred, the dsRNA may contain single or multiple base-pair random mismatches between the RNA and the target 3'-UTR.

As used herein, the term "portion" refers to a nucleic acid sequence which is a fragment of a target gene, and which includes at least part of an exon sequence of the target gene. Preferably a "portion" is about 30 consecutive residues of the target gene in length, and includes 5, 10, 15, 20, 25, and up to 30 consecutive residues or more of the target gene.

As used herein, the term "comprising" or "involving" "a phosphodiester bond" or "phosphodiester group" refers to a chemical entity comprising a group of general structural formula I

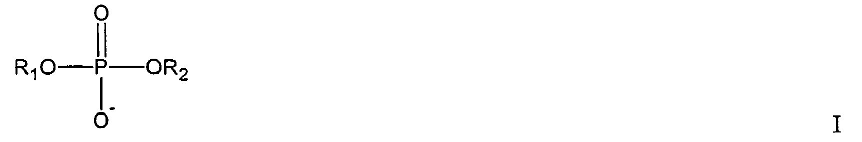

Therein, R₁O stands for a 5'-end of a dsRNA, and R₂ for a lipophilic group. However, the oxygen atoms in I may be replaced by any other suitable element, for example, to increase the stability of the overall construct.

As used herein, the term "treatment" refers to the application or administration of a therapeutic agent to a patient, or application or administration of a therapeutic agent to an isolated tissue or cell line from a patient, who has a disorder, e.g., a disease or condition, a symptom of disease, or a predisposition toward a disease, with the purpose to cure, heal, alleviate, relieve, alter, remedy, ameliorate, improve, or affect the disease, the symptoms of disease, or the predisposition toward disease.

As used herein, a "pharmaceutical composition" comprises a pharmacologically effective amount of a dsRNA and a pharmaceutically acceptable carrier. As used herein, "pharmacologically effective amount," "therapeutically effective amount" or simply "effective amount" refers to that amount of an RNA effective to produce the intended pharmacological, therapeutic or preventive result. For example, if a given clinical treatment is considered effective when there is at least a 25% reduction in a measurable parameter associated with a disease or disorder, a therapeutically effective amount of a drug for the treatment of that disease or disorder is the amount necessary to effect at least a 25% reduction in that parameter.

The term "pharmaceutically acceptable carrier" refers to a carrier or diluent for administration of a therapeutic agent. Pharmaceutically acceptable carriers for therapeutic use are well known in the pharmaceutical art, and are described, for example, in *Remington's Pharmaceutical Sciences,* Mack Publishing Co. (A.R. Gennaro, ed. 1985). Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

As used herein, a "transformed cell" is a cell into which a dsRNA molecule has been introduced by means of recombinant DNA techniques.

As used herein, the term "lipophilic" or "lipophilic group" broadly refers to any compound or chemical moiety having an affinity for lipids. Lipophilicity is often expressed in terms of the octanol-water partition coefficient, logK_{ow}, where K_{ow} is the ratio of the ratio of a chemical's concentration in the octanol-phase to its concentration in the aqueous phase of a two-phase system at equilibrium. The octanol-water partition coefficient is a laboratory-measured property of a substance. However, it may also be predicted by using coefficients attributed to the structural components of a chemical which are calculated using first-principle or empirical methods (see, for example, Tetko, I.V., et al., J Chem Inf Comput Sci. (2001) 41:1407-21). It provides a thermodynamic measure of the tendency of the substance to prefer a non-aqueous or oily milieu rather than water (i.e. its hydrophilic/lipophilic balance). In principle, a chemical substance is lipophilic in character when its logK_{ow} exceeds 0. However, for the purposes of the instant invention, for a lipophilic group R₂, as defined above, the parent compound R₂-OH will possess a logK_{ow} in excess of 1, preferably in excess of 1.5, more preferably in excess of 2, yet more preferably in excess of 3 or 4, and most preferably in excess of 5; herein, where it is referred to the logK_{ow} of a lipophilic group, it shall be understood to mean the logK_{ow} of the parent compound R₂-OH. The predicted logK_{ow} of 6-amino hexanol, the 5'-end substituent used in the investigations by Schwarz, D.S. et al (Mol. Cell (2002) 10:537-548) is predicted as approximately 0.7. Other investigations used substituents with considerably lower logK_{ow}. Using the same method, the logK_{ow} of cholesteryl N-(hexan-6-ol) carbamate is predicted as 10.7.

For the avoidance of doubt, in the determination of logK_{ow} for a given lipophilic group hereunder, values determined experimentally shall prevail over values obtained from prediction algorithms. Where an experimental determination is for any reason not feasible, the prediction method of Tetko, LV., et al. (J Chem Inf Comput Sci. (2001) 41:1407-21) shall be employed.

### II. Double-stranded ribonucleic acid (dsRNA)

In one embodiment, the invention relates to a double-stranded ribonucleic acid (dsRNA) having a nucleotide sequence which is substantially identical to at least a portion of a target gene, such as a 3'-UTR of a (+) strand RNA virus. The dsRNA comprises two RNA strands that are sufficiently complementary to hybridize to form the duplex structure. One strand of the dsRNA comprises the nucleotide sequence that is substantially identical to a portion of the target gene (the "sense" strand), and the other strand (the "complementary" or "antisense" strand) comprises a sequence that is complementary to the target gene. The dsRNA of the present invention further comprises one lipophilic group. Lipophilic groups encompass compounds of many different types, including those having aromatic, aliphatic or alicyclic characteristics, and combinations thereof In one embodiment, the invention provides a double stranded RNA molecule comprising a complementary RNA strand and one lipophilic group, wherein the complementary RNA strand has a nucleotide sequence which is complementary to a target RNA and where the target RNA is an mRNA transcript of a portion of a target gene or a portion of a (+) strand RNA virus, wherein the lipophilic group is covalently linked to the 5' end of the sense strand. In one embodiment, the lipophilic group is covalently linked to the 5' end of the sense strand by a covalent linkage which does not comprise a phosphodiester group.

In specific embodiments, the lipophilic group is an aliphatic, alicyclic, or polyalicyclic substance, such as a steroid (e.g., sterol) or a branched aliphatic hydrocarbon. The lipophilic group generally comprises a hydrocarbon chain, which may be cyclic or acyclic. The hydrocarbon chain may comprise various substituents and/or at least one heteroatom, such as an oxygen atom. Such lipophilic aliphatic moieties include, without limitation, saturated or unsaturated fatty acids, waxes (e.g., monohydric alcohol esters of fatty acids and fatty diamides), terpenes (e.g., the C₁₀ terpenes, C₁₅ sesquiterpenes, C₂₀ diterpenes, C₃₀ triterpenes, and C₄₀ tetraterpenes), and other polyalicyclic hydrocarbons. The lipophilic group may be attached by any method known in the art, including via a functional grouping present in or introduced into the dsRNA, such as a hydroxy group (e.g., --CO-CH₂-OH). Conjugation of the dsRNA and the lipophilic group may occur, for example, through formation of an ether or a carboxylic or carbamoyl ester linkage between the hydroxy and an alkyl group R--, an alkanoyl group RCOor a substituted carbamoyl group RNHCO-. The alkyl group R may be cyclic (e.g., cyclohexyl) or acyclic (e.g., straight-chained or branched; and saturated or unsaturated). Alkyl group R may be a butyl, pentyl, hexyl, heptyl, octyl, nonyl, decyl, undecyl, dodecyl, tridecyl, tetradecyl, pentadecyl, hexadecyl, heptadecyl or octadecyl group, or the like. Preferably, the lipophilic group is conjugated to the 5'-hydroxyl group of the terminal nucleotide. In a preferred embodiment, the liphophilic group is 12-hydroxydodeconoic acid bisdecylamide.

In another embodiment, the lipophilic group is a steroid, such as sterol. Steroids are polycyclic compounds containing a perhydro-1,2-cyclopentanophenanthrene ring system. Steroids include, without limitation, bile acids (e.g., cholic acid, deoxycholic acid and dehydrocholic acid), cortisone, digoxigenin, testosterone, cholesterol and cationic steroids, such as cortisone. A "cholesterol derivative" refers to a compound derived from cholesterol, for example by substitution, addition or removal of substituents. The steroid may be attached to the dsRNA by any method known in the art, for example, as described in Section III below. In a preferred embodiment, the liphophilic group is cholesteryl (6-hydroxyhexyl) carbamate.

In another embodiment, the lipophilic group is an aromatic moiety. In this context, the term "aromatic" refers broadly to mono- and polyaromatic hydrocarbons. Aromatic groups include, without limitation, C₆ - C₁₄ aryl moieties comprising one to three aromatic rings, which may be optionally substituted; "aralkyl" or "arylalkyl" groups comprising an aryl group covalently linked to an alkyl group, either of which may independently be optionally substituted or unsubstituted; and "heteroaryl" groups. As used herein, the term "heteroaryl" refers to groups having 5 to 14 ring atoms, preferably 5, 6, 9, or 10 ring atoms; having 6,10, or 14 π electrons shared in a cyclic array; and having, in addition to carbon atoms, between one and about three heteroatoms selected from the group consisting of nitrogen (N), oxygen (O), and sulfur (S).

As employed herein, a "substituted" alkyl, cycloalkyl, aryl, heteroaryl, or heterocyclic group is one having between one and about four, preferably between one and about three, more preferably one or two, non-hydrogen substituents. Suitable substituents include, without limitation, halo, hydroxy, nitro, haloalkyl, alkyl, alkaryl, aryl, aralkyl, alkoxy, aryloxy, amino, acylamino, alkylcarbamoyl, arylcarbamoyl, aminoalkyl, alkoxycarbonyl, carboxy, hydroxyalkyl, alkanesulfonyl, arenesulfonyl, alkanesulfonamido, arenesulfonamido, aralkylsulfonamido, alkylcarbonyl, acyloxy, cyano, and ureido groups.

The complementary RNA strand is less than 30 nucleotides, preferably between 16 and 30 nucleotides, more preferably between 16 and 25 nucleotides, and most preferably between 20 and 25 nucleotides in length. The dsRNA can be synthesized by standard methods known in the art, e.g., by use of an automated DNA synthesizer, such as are commercially available from Biosearch, Applied Biosystems, Inc. In a particular embodiment, the antisense (complementary) RNA strand comprises the sequence set forth in SEQ ID NO:1, and the sense RNA strand comprises the sequence set forth in SEQ ID NO:2.

In one embodiment, at least one end of the dsRNA is blunt. dsRNA with at least one blunt end show improved stability as compared to dsRNA having two nucleotide overhangs. dsRNA with at least one blunt end shows greater in vivo stability (i.e., is more resistant to degradation in the blood, plasma, and cells). However, dsRNAs having at least one nucleotide overhang have superior inhibitory properties than their blunt-ended counterparts. The presence of only one nucleotide overhang strengthens the interference activity of the dsRNA, without effecting its overall stability. dsRNA having only one overhang has proven particularly effective in vivo (as well as in a variety of cells, and cell culture mediums), and are more stable than dsRNA having two blunt ends. The single-stranded nucleotide overhang may be 1 to 4, preferably 1 or 2, nucleotides in length. Preferably, the single-stranded overhang is located at the 3'-end of the complementary (antisense) RNA strand. Such dsRNAs have improved stability and inhibitory activity, thus allowing administration at low dosages, i.e., less than 5 mg/kg body weight of the recipient per day. Preferably, the complementary strand of the dsRNA has a nucleotide overhang at the 3'-end, and the 5'-end is blunt.

In another embodiment, the lipophilic group is covalently linked to the 5'-end of either the complementary RNA strand or the second (sense) RNA strand. dsRNA having a covalently linked lipophilic group at a 5'-end of an RNA strand can be easily synthesized, for example by solid-phase synthesis, as described elsewhere herein. The dsRNA has only one pendant lipophilic group, which is covalently linked to the 5'-end of the second (sense) RNA strand. In one embodiment, the covalent linkage comprises a phosphodiester-bond. In another embodiment, the lipophilic group is linked to the 5'-terminus of the sense strand, and the covalent linkage does not comprise a phosphodiester bond. In yet another embodiment, the lipophilic group is covalently attached to the 5'-end of the complementary strand, and the linkage comprises a phosphodiester group.

dsRNA with one covalently linked lipophilic group show improved RNA interference activity as compared to the corresponding (unmodified) dsRNA without an attached lipophilic group. Moreover, the present inventors have discovered that dsRNA comprising a covalently linked lipophilic group can be taken up by cells with or without a transfection aid, such as the transfection reagent sold under the tradename FuGENE 6^{™} (Roche Diagnostics GmbH, Sandhofer Str. 116, D-68305, Mannheim, Germany), and that these derivatized dsRNA show surprisingly improved activity regardless of the mechanism of entry into the cell. Thus, unlike similarly conjugated antisense RNA, the improved inhibitory activity of the dsRNA of the present invention is independent of cellular association or receptor binding, and thus is not a consequence of enhanced transport across cell membranes.

It is currently believed by the skilled person that dsRNA with 5'-modifications in the antisense strand is unable to cause RNA interference (Nykänen et al., Cell (2001) 107:309-321, Schwarz, D.S., et al., Mol. Cell (2002) 10:537-548, Chiu, Y.L., and Rana, T.M., Mol. Cell (2002) 10:549-561, Czauderna, F., et al., Nucleic Acids Research (2003) 31:2705-2716). It is the merit of the instant inventors to prove that 5'-modifications where the modifying group is linked to the 5'-end of the antisense strand via a phosphodiester group may very well function as RNA interference agents. Conversely, where the modifying group is linked to the 5'-end of the antisense strand via a different linkage, RNA interference is indeed abolished. However, this effect may be used to avoid side effects stemming from the sense strand of a dsRNA designed to inhibit one gene acting as an antisense strand for another gene which it is fortuitously complementary to. Where the sense strand is 5'-linked to a modifying group by a linkage not comprising a phosphodiester group, the dsRNA may not any more as inhibitor of the expression of genes complementary or partially complementary to its sense strand.

A linkage not comprising a phosphodiester group may be any linkage not comprising a phosphodiester group. For example, without limitation, a non-phosphodiester group comprising linkage may be effected via an ester group of a carboxylic acid, an amide group, an ether group, a thioether group, an amino group, a linear aliphatic chain, a branched aliphatic chain, a polyether chain, a cyclic group or an aromatic group. Preferably, the linkage is not easily cleaved by enzymes present within a mammalian cell.

### III. Method of Making dsRNA Having Improved Biological Activity

The invention further relates to a method for making a dsRNA having improved RNA interference activity. The method comprises synthesizing a first (complementary) RNA strand and a second (sense) RNA strand, wherein one of the RNA strands comprises a pendant lipophilic group, and mixing the first and second RNA strands to form a dsRNA. The step of synthesizing the RNA strand preferably involves solid-phase synthesis, wherein individual nucleotides are joined end to end through the formation of internucleotide 3'-5' phosphodiester bonds in consecutive synthesis cycles.

In one embodiment, a lipophilic molecule having a phosphoramidite group is coupled to the 5'-end of either the first (complementary) or second (sense) RNA strand in the last synthesis cycle. In the solid-phase synthesis of an RNA, which is described in more detail below, the nucleotides are initially in the form of nucleoside phosphoramidites. In each synthesis cycle, a further nucleoside phosphoramidite is linked to the 5'-OH group of the previously incorporated nucleotide. If the lipophilic molecule has a phosphoramidite group, it can be coupled in a manner similar to a nucleoside phosphoramidite to the free 5'-OH end of the RNA synthesized previously in the solid-phase synthesis. The synthesis can take place in an automated and standardized manner using a conventional RNA synthesizer, as described below. Synthesis of the lipophilic molecule having the phosphoramidite group may include phosphitylation of a free hydroxyl to generate the phosphoramidite group. Synthesis of a lipophilic molecule comprising a phosphoramidite group may involve conversion of cholesteryl chloroformate into an amide, or the reaction of 12-hydroxylauric acid with di-n-decylamine to form an amide linkage. In the exemplified embodiments, the lipophilic molecule having a phosphoramidite group is cholesteryl N-[6-(2-cyanoethoxy)-N,N-diisopropylaminophosphanyloxy]-hexyl carbamate or 12-[(2-cyanoethoxy) N,N-diisopropylamino-phosphanyloxy]dodecanoic acid bisdecylamide.

In one specific embodiment of the instant invention, the lipophilic molecule is attached to a 5'-end of the sense strand, wherein the linkage of the lipophilic molecule does not comprise a phosphodiester group. The synthesis of a sense strand bearing a 5'-derivatization wherein the linkage does not comprise a phosphodiester bond is equally easily achieved using standard methods of synthesis known to the skilled person. For example, the nucleotide part of the strand may be synthesized using the standard phosporamidite chemistry mentioned above, and the deprotected terminal 5'-OH of the strand may be reacted with a carboxylic acid halide, such as, for example, pivaloyl chloride. The skilled person will know many other methods by which a linkage not comprising a phosphodiester group can be established.

In general, the oligonucleotides of the present can be synthesized using protocols known in the art, for example, as described in Caruthers, et al., Methods in Enzymology (1992) 211:3-19; Thompson, et al., International PCT Publication No. WO 99/54459; Wincott, et al., Nucl. Acids Res. (1995) 23:2677-2684; Wincott, et aL, Methods Mol. Bio., (1997) 74:59; Brennan, et al., Biotechnol. Bioeng. (1998) 61:33-45; and Brennan, U.S. Pat. No. 6,001,311. In general, the synthesis of oligonucleotides involves conventional nucleic acid protecting and coupling groups, such as dimethoxytrityl at the 5'-end, and phosphoramidites at the 3'-end. In a non-limiting example, small scale syntheses are conducted on a Expedite 8909 RNA synthesizer sold by Applied Biosystems, Inc. (Weiterstadt, Germany), using ribonucleoside phosphoramidites sold by ChemGenes Corporation (Ashland Technology Center, 200 Homer Avenue, Ashland, MA 01721, USA). Alternatively, syntheses can be performed on a 96-well plate synthesizer, such as the instrument produced by Protogene (Palo Alto, Calif., USA), or by methods such as those described in Usman, et al., J. Am. Chem. Soc. (1987) 109:7845; Scaringe, et al., Nucl. Acids Res. (1990) 18:5433; Wincott, et al., Nucl. Acids Res. (1990) 23:2677-2684; and Wincott, et al., Methods Mol. Bio. (1997) 74:59.

The nucleic acid molecules of the present invention may be synthesized separately and joined together post-synthetically, for example, by ligation (Moore, et al., Science (1992) 256:9923; Draper, et al., International PCT publication No. WO 93/23569; Shabarova, et al., Nucl. Acids Res. (1991) 19:4247; Bellon, et al., Nucleosides & Nucleotides (1997) 16:951; Bellon, et al., Bioconjugate Chem. (1997) 8:204; or by hybridization following synthesis and/or deprotection. The nucleic acid molecules can be purified by gel electrophoresis using conventional methods or can be purified by high pressure liquid chromatography (HPLC; see Wincott et al., supra) and resuspended in water.

### IV. Pharmaceutical compositions comprising dsRNA

In one embodiment, the invention relates to a pharmaceutical composition comprising a dsRNA, as described in the preceding section, and a pharmaceutically acceptable carrier, as described below. The pharmaceutical composition comprising the dsRNA is useful for treating a disease caused by expression of a target gene. In this aspect of the invention, the dsRNA of the invention is formulated as described below. The pharmaceutical composition is administered in a dosage sufficient to inhibit expression of the target gene.

The pharmaceutical compositions of the present invention are administered in dosages sufficient to inhibit the expression or activity of the target gene, for example, the activity or replication of a (+) strand RNA virus, such as HCV. Compositions comprising the dsRNA of the invention can be administered at surprisingly low dosages. A maximum dosage of 5 mg dsRNA per kilogram body weight per day may be sufficient to inhibit or completely suppress the activity or replication of the target virus.

In general, a suitable dose of dsRNA will be in the range of 0.01 to 5.0 milligrams per kilogram body weight of the recipient per day, preferably in the range of 0.1 to 2.5 milligrams per kilogram body weight of the recipient per day, more preferably in the range of 0.1 to 200 micrograms per kilogram body weight per day, and most preferably in the range of 0.1 to 100 micrograms per kilogram body weight per day. The pharmaceutical composition may be administered once daily, or the dsRNA may be administered as two, three, four, five, six or more sub-doses at appropriate intervals throughout the day. In that case, the dsRNA contained in each sub-dose must be correspondingly smaller in order to achieve the total daily dosage. The dosage unit can also be compounded for delivery over several days, e.g., using a conventional sustained release formulation which provides sustained release of the dsRNA over a several day period. Sustained release formulations are well known in the art. In this embodiment, the dosage unit contains a corresponding multiple of the daily dose.

The skilled artisan will appreciate that certain factors may influence the dosage and timing required to effectively treat a subject, including but not limited to the severity of the infection or disease, previous treatments, the general health and/or age of the subject, and other diseases present. Moreover, treatment of a subject with a therapeutically effective amount of a composition can include a single treatment or a series of treatments. Estimates of effective dosages and in vivo half-lives for the individual dsRNAs encompassed by the invention can be made using conventional methodologies or on the basis of in vivo testing using an appropriate animal model, as described elsewhere herein.

Advances in mouse genetics have generated a number of mouse models for the study of various human diseases. For example, mouse repositories can be found at The Jackson Laboratory, Charles River Laboratories, Taconic, Harlan, Mutant Mouse Regional Resource Centers (MMRRC) National Network and at the European Mouse Mutant Archive. Such models may be used for in vivo testing of dsRNA, as well as for determining a therapeutically effective dose.

The pharmaceutical compositions encompassed by the invention may be administered by any means known in the art including, but not limited to oral or parenteral routes, including intravenous, intramuscular, intraperitoneal, subcutaneous, transdermal, airway (aerosol), rectal, vaginal and topical (including buccal and sublingual) administration. In preferred embodiments, the pharmaceutical compositions are administered by intravenous or intraparenteral infusion or injection.

For oral administration, the dsRNAs useful in the invention will generally be provided in the form of tablets or capsules, as a powder or granules, or as an aqueous solution or suspension.

Tablets for oral use may include the active ingredients mixed with pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredients is mixed with water or an oil such as peanut oil, liquid paraffin or olive oil.

For intramuscular, intraperitoneal, subcutaneous and intravenous use, the pharmaceutical compositions of the invention will generally be provided in sterile aqueous solutions or suspensions, buffered to an appropriate pH and isotonicity. Suitable aqueous vehicles include Ringer's solution and isotonic sodium chloride. In a preferred embodiment, the carrier consists exclusively of an aqueous buffer. In this context, "exclusively" means no auxiliary agents or encapsulating substances are present which might affect or mediate uptake of dsRNA in the cells that harbor the virus. Such substances include, for example, micellar structures, such as liposomes or capsids, as described below. Surprisingly, the present inventors have discovered that compositions containing only naked dsRNA and a physiologically acceptable solvent are taken up by cells, where the dsRNA effectively inhibits replication of the virus. Although microinjection, lipofection, viruses, viroids, capsids, capsoids, or other auxiliary agents are required to introduce dsRNA into cell cultures, surprisingly these methods and agents are not necessary for uptake of dsRNA in vivo. The dsRNA of the present invention are particularly advantageous in that they do not require the use of an auxiliary agent to mediate uptake of the dsRNA into the cell, many of which agents are toxic or associated with deleterious side effects. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

The pharmaceutical compositions useful according to the invention also include encapsulated formulations to protect the dsRNA against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811; PCT publication WO 91/06309; and European patent publication EP-A-43075.

Toxicity and therapeutic efficacy of dsRNAs can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred.

The data obtained from cell culture assays and animal studies can be used in formulation a range of dosage for use in humans. The dosage of compositions of the invention lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range of the compound or, when appropriate, of the polypeptide product of a target sequence (e.g., achieving a decreased concentration of the polypeptide) that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

In addition to their administration individually or as a plurality, as discussed above, the dsRNAs useful according to the invention can be administered in combination with other known agents effective in treating viral infections and diseases. In any event, the administering physician can adjust the amount and timing of dsRNA administration on the basis of results observed using standard measures of efficacy known in the art or described herein.

For oral administration, the dsRNAs useful in the invention will generally be provided in the form of tablets or capsules, as a powder or granules, or as an aqueous solution or suspension.

### V. Methods for treating diseases caused by expression of a target gene.

In a method for treating a subject having a disease or at risk of developing a disease caused by the expression of a target gene the dsRNA can act as novel therapeutic agents for controlling one or more of cellular proliferative and/or differentiative disorders, disorders associated with bone metabolism, immune disorders, hematopoietic disorders, cardiovascular disorders, liver disorders, viral diseases, or metabolic disorders. The method comprises administering a pharmaceutical composition of the invention to the patient (e.g., human), such that expression of the target gene is silenced. Because of their high efficiency and specificity, the dsRNAs of the present invention specifically target mRNAs of target genes of diseased cells and tissues, as described below, and at surprisingly low dosages. The pharmaceutical compositions are formulated as described in the preceding section, which is hereby incorporated by reference herein.

In the prevention of disease, the target gene may be one which is required for initiation or maintenance of the disease, or which has been identified as being associated with a higher risk of contracting the disease. In the treatment of disease, the dsRNA can be brought into contact with the cells or tissue exhibiting the disease. For example, dsRNA substantially identical to all or part of a mutated gene associated with cancer, or one expressed at high levels in tumor cells, e.g. aurora kinase, may be brought into contact with or introduced into a cancerous cell or tumor gene.

Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast and liver origin. As used herein, the terms "cancer," "hyperproliferative," and "neoplastic" refer to cells having the capacity for autonomous growth, i.e., an abnormal state of condition characterized by rapidly proliferating cell growth. These terms are meant to include all types of cancerous growths or oncogenic processes, metastatic tissues or malignantly transformed cells, tissues, or organs, irrespective of histopathologic type or stage of nvasiveness. Proliferative disorders also include hematopoietic neoplastic disorders, including diseases involving hyperplastic/neoplatic cells of hematopoietic origin, e.g., arising from myeloid, lymphoid or erythroid lineages, or precursor cells thereof.

The pharmaceutical compositions of the present invention can also be used to treat a variety of immune disorders, in particular those associated with overexpression of a gene or expression of a mutant gene. Examples of hematopoietic disorders or diseases include, without limitation, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, automimmune thyroiditis, dermatitis (including atopic dermatitis and eczematous dermatitis), psoriasis, Sjogren's Syndrome, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing, loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft versus-host disease, cases of transplantation, and allergy.

dsRNAs of the invention are prepared as described herein to target expressed sequences of a virus, thus ameliorating viral activity and replication. The molecules can be used in the treatment and/or diagnosis of viral infected tissue, both animal and plant. Also, such molecules can be used in the treatment of virus-associated carcinoma, such as hepatocellular cancer.

Because of their high specificity and activity in cells infected by HCV (e.g., hepatocytes), the dsRNAs of the present invention are particularly useful for targeting (+) strand RNA viruses having a 3'-UTR, as described above, and at surprisingly low dosages.

Examples of (+) strand RNA viruses which can be targeted for inhibition include, without limitation, picornaviruses, caliciviruses, nodaviruses, coronaviruses, arteriviruses, flaviviruses, and togaviruses. Examples of picomaviruses include enterovirus (poliovirus 1), rhinovirus (human rhinovirus 1A), hepatovirus (hepatitis A virus), cardiovirus (encephalomyocarditis virus), aphthovirus (foot-and-mouth disease virus 0), and parechovirus (human echovirus 22). Examples of caliciviruses include vesiculovinis (swine vesicular exanthema virus), lagovirus (rabbit hemorrhagic disease virus), "Norwalk-like viruses" (Norwalk virus), "Sapporo-like viruses" (Sapporo virus), and "hepatitis E-like viruses" (hepatitis E virus). Betanodavirus (striped jack nervous necrosis virus) is the representative nodavirus. Coronaviruses include coronavirus (avian infections bronchitis virus) and torovirus (Berne virus). Arterivirus (equine arteritis virus) is the representative arteriviridus. Togavirises include alphavirus (Sindbis virus) and rubivirus (Rubella virus). Finally, the flaviviruses include flavivirus (Yellow fever virus), pestivirus (bovine diarrhea virus), and hepacivirus (hepatitis C virus). In a preferred embodiment, the virus is hepacivirus, the hepatitis C virus. Although the foregoing list exemplifies vertebrate viruses, the present invention encompasses the compositions for treating infections and diseases caused by any (+) strand RNA virus having a 3'-UTR, regardless of the host. For example, the invention encompasses the treatment of plant diseases caused by sequiviruses, comoviruses, potyviruses, sobemovirus, luteoviruses, tombusviruses, tobavirus, tobravirus, bromoviruses, and closteroviruses.

The pharmaceutical compositions encompassed by the invention may be administered by any means known in the art including, but not limited to oral or parenteral routes, including intravenous, intramuscular, intraperitoneal, subcutaneous, transdermal, airway (aerosol), rectal, vaginal and topical (including buccal and sublingual) administration. In preferred embodiments, the pharmaceutical compositions are administered by intravenous or intraparenteral infusion or injection.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

In addition, the materials, methods, and examples are illustrative only and not intended to be limiting.

### EXAMPLES

### Example 1: Synthesis of a cholesterol derivative

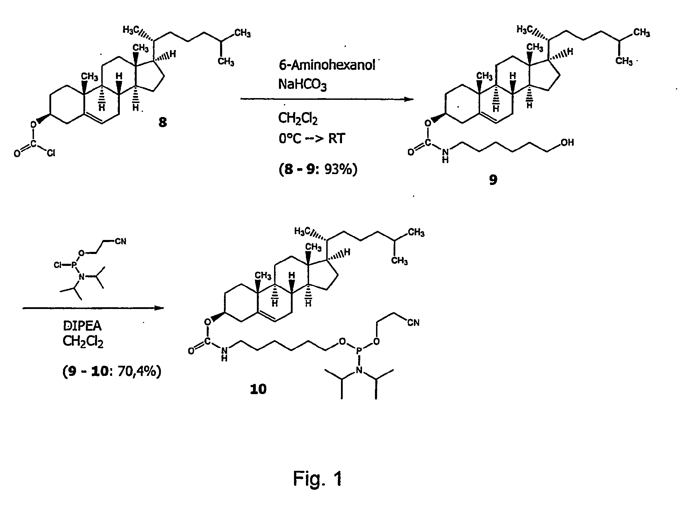

The synthetic scheme for preparing an exemplified cholesterol derivative, referred to herein as "Chol," is shown in FIG. 1.

In this synthetic scheme, cholesteryl chloroformate 8 is converted as follows by adding 1.2 equivalents of 6-aminohexanol in the presence of NaHCO₃ into the amide cholesteryl (6-hydroxy-hexyl) carbamate 9: 2 g (4.45 mmol) of cholesteryl chloroformate 8 were dissolved in 10 ml of CH₂Cl₂, and 561 mg (6.68 mmol) of NaHCO₃ were added. Then, while stirring vigorously at 0°C, 626 mg (5.34 mmol) of 6-aminohexanol dissolved in 10 ml of CH₂Cl₂ were added. The drop rate during this was about 1 drop per second. The reaction took place over 16 hours, during which the temperature slowly rose to room temperature. The reaction mixture was then diluted with 30 ml of CH₂Cl₂ and extracted with H₂O, and the aqueous phase was back-extracted with CH₂Cl₂. The combined organic phases were then dried over Na₂SO₄, filtered and concentrated. The crude product obtained in this way was purified by column chromatography, using a column with a diameter of 4.5 cm and packed to a height of 15 cm with silica gel, particle size 40-63 µm (Merck KGaA, Frankfurter Str. 250, Darmstadt, Germany). Cyclohexane/ethyl acetate 2:1 was used as eluent.

The free hydroxyl group of the resulting product 9 was phosphitylated as follows, i.e. converted into a phosphoramidite group: 500 mg (0.944 mmol) of the precursor 9 were dissolved in 15 ml of anhydrous CH₂Cl₂ and, in a countercurrent of argon, put into a flask which had previously been evacuated and ventilated with argon. Then, while stirring, 485 µl (2.83 mmol) of *N,N-*diisopropylethylamine (DIPEA) and, dropwise, 261 µl (1.04 mmol) of 2-cyanoethyl diisopropyl-chlorophosphoramidite were added, and the reaction mixture was stirred in a stream of argon at room temperature. The reaction was complete after 90 minutes. The reaction mixture was first diluted with CH₂Cl₂ and then extracted with saturated NaCl solution. Finally, the aqueous phase was back-extracted with CH₂Cl₂. The combined organic phases were then dried over Na₂SO₄, filtered and concentrated using a rotary evaporator ventilated with argon. The crude product was purified by column chromatography under argon pressure, using a column with a diameter of 4.5 cm and packed to a height of 8 cm with silica gel, particle size 40-63 µm (Merck KGaA). Cyclohexane/ethyl acetate 6:1 + 1% triethylamine (Et₃N) was used as eluent. The silica gel used in this case was previously slurried with cyclohexane/1% Et₃N for 2 hours. The product obtained was cholesteryl N-[6-(2-cyanoethoxy)-N,N-diisopropylaminophosphanyloxy]-hexyl carbamate 10, which is referred to herein as "Chol".

### Example 2: Synthesis of a di-n-decylamine derivative

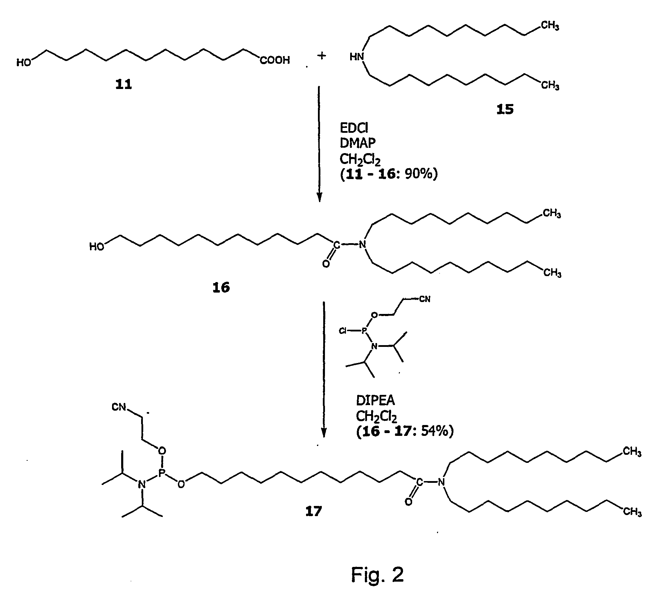

The synthetic scheme for preparing an exemplified di-n-decylamine derivative, referred to herein as "C32," is shown in FIG. 2.

To synthesize C32, 12-hydroxylauric acid 11 was reacted as follows with 2 equivalents of the secondary amine di-n-decylamine 15 to give the product 12-hydroxydodecanoic acid bisdecylamide 16: 1 g (4.623 mmol) of 12-hydroxylauric acid 11 was reacted with 282 mg (2.31 mmol) of p*-N,N*-dimethylaminopyridine (DMAP) and 1.329 g (6.94 mmol) of *N-*(3-dimethylaminopropyl)-*N'*-ethylcarbodiimide hydrochloride (EDC1). Then 2.751 g (9.246 mmol) of di-n-decylamine 15 were added, and the reaction mixture was stirred vigorously at room temperature. After 16 hours, the crude product was diluted with CH₂Cl₂ and extracted twice with H₂O, during which a flocculant white solid formed in the aqueous phase. The aqueous phase was back-extracted with CH₂Cl₂. The organic phases were then dried with Na₂SO₄, filtered and concentrated. The crude product obtained in this way was purified by column chromatography. A column with a diameter of 4.5 cm and packed to a height of 19 cm with silica gel, particle size 40-63 µm (Merck KgaA), was used for this. Cyclohexane/ethyl acetate 4:1 was used as eluent.

The free hydroxyl group of the resulting product 16 was phosphitylated as follows: 1.018 g (2.05 mmol) of the amide 16 were dissolved in 30 ml of anhydrous CH₂Cl₂ and put, in a countercurrent of argon, into a flask which had previously been evacuated and ventilated with argon. Then, while stirring, 1 054 µl (6.16 mmol) of DIPEA and, dropwise, 504 µl (2.26 mmol) of 2-cyanoethyl diisopropylchloro-phosphoramidite were added, and the reaction mixture was stirred in a stream of argon at room temperature. The reaction was complete after 2 hours. The reaction mixture was initially diluted with CH₂Cl₂ and then extracted with saturated NaCl solution. Finally, the aqueous phase was back-extracted with CH₂Cl₂. The combined organic phases were then dried over Na₂SO₄, filtered and concentrated using a rotary evaporator ventilated with argon. The crude product was purified by column chromatography under argon pressure, using a column with a diameter of 4.5 cm and packed to a height of 20 cm with silica gel, particle size 40-63 µm (Merck KGaA). Cyclohexane/ethyl acetate 8:1 + 1% Et₃N was used as eluent. The silica gel used was previously slurried with cyclohexane/1% Et₃N for 2 hours. The product obtained was 12-[(2-cyanoethoxy) -N,N-diisopropylaminophosphanyloxy]dodecanoic acid bisdecylamide 17, which is referred to herein as "C32."

The cholesterol and di-n-decylamine derivatives, Chol and C32, were synthesized using chemicals obtained from Fluka, Industriestrasse 25, CH-9471 Buchs SG, Switzerland, and chemicals for eluents for column chromatography were obtained from Carl Roth GmbH & Co, Schoemperlenstr. 1-5, 76185 Karlsruhe, Germany.

### Example 3: RNA synthesis

Sequences from the highly conserved 3'-UTR region of the hepatitis C virus (HCV) gene and the β-galactosidase (β-Ga1) gene from *E. coli* were selected as target sequences for this experiment. The following double-stranded oligoribonucleotide sequences, designated SEQ ID NOS:1-4 in the sequence listing, were used in the transfection experiments:
HCV: dsRNA whose complementary RNA strand is complementary to a target sequence from the HCV gene:
   S2: 5'- ACG GCU AGC UGU GAA AGG UCC- 3' (HCV11s - SEQ ID NO:2)
   S1: 3'-AG UGC CGA UCG ACA CUU UCC AGG- 5' (HCV2 - SEQ ID NO:1)
Gal: dsRNA whose complementary RNA strand is complementary to a target sequence from the β-galactosidase gene:
   S2: 5'- GUG AAA UUA UCG AUG AGC GUG- 3' (Gal3s - SEQ ID NO:4)
   S1: 3'-GC CAC UUU AAU AGC UAC UCG CAC- 5' (Gal2 - SEQ ID NO:3)

A dsRNA referred to herein as "K22," which is not expressed and has the sequences of SEQ ID Nos:5 and 6, was used as a negative control.

The RNA single strands were prepared using an RNA synthesizer (Expedite 8909 type, Applied Biosystems, Weiterstadt, Germany) and conventional solid-phase synthesis using ribonucleoside phosphoramidites (ChemGenes Corporation, Ashland Technology Center, 200 Homer Avenue, Ashland, MA 01721, USA). The amidites cholesteryl [6- (2-cyanoethoxy) - N,N-diisopropylamino-phosphanyloxy]carbamate 10 and 12-[(2-cyanoethoxy) -N,N-diisopropylamino-phosphanyloxy]dodecanoic acid bisdecylamide 17 were coupled to the 5'-OH end of the synthesized RNA, in manner analogous to the ribonucleoside phosphoramidites, in the last synthetic cycle.

RNA single strands with and without a lipophilic group were purified by HPLC, using NucleoPac PA-100, 9 x 250 mm from Dionex GmbH, Am Wörtzgarten 10, 65510 Idstein, Germany; low-salt buffer employed: 20 mM Tris, 10 mM NaClO₄, pH 6.8, 6 M urea; high-salt buffer employed 20 mM Tris, 400 mM NaClO₄, pH 6.8, 6 M urea. The flow rate was 3 ml/minute. The hybridization of the single strands to give the double strand took place by heating the stoichiometric mixture of the single strands in 10 mM sodium phosphate buffer, pH 6.8, 100 mM NaCl, at 80-90°C and subsequent slow cooling to room temperature over 6 hours.

The following lipophilic derivatives of dsRNA (or siRNA) were synthesized:
Gal with the lipophilic group Chol on strand S 1 (complementary RNA strand): GalChol-as
Gal with the lipophilic group Chol on strand S2 (sense strand): GalChol-s
Gal with the lipophilic group Chol on each of strand S 1 and strand S2: GalChol-2
Gal with the lipophilic group C32 on strand S1: Ga1C32-as Gal with the lipophilic group C32 on strand S2: Ga1C32-s
Gal with the lipophilic group C32 on each of strand S1 and strand S2: Ga1C32-2
HCV with the lipophilic group Chol on strand S1: HCVCho1-as
HCV with the lipophilic group Chol on strand S2: HCVCho1-s
HCV with the lipophilic group Chol on each of strand S1 and strand S2: HOVCho1-2
HCV with the lipophilic group C32 on strand S1: HCVC32-as
HCV with the lipophilic group C32 on strand S2: HCVC32-s
HCV with the lipophilic group C32 on each of strand S1 and strand S2: HCVC32-2

### Example 4: Inhibition of viral gene expression using dsRNA

The inhibitory effect of dsRNA of the invention on expression and activity of viral genes was evaluated using a non-pathogenic substitute system. Specifically, an oligonucleotide as shown in SEQ ID NO:7 of the sequence listing was cloned in front of a gene coding for E. coli β-galactosidase from the commercially available expression vector pβGal-Control (BD Biosciences Clontech, Tullastrasse 4, D-69126 Heidelberg, Germany, Gene Accession Number U13186; nucleotide 280-3429). SEQ ID NO:7 corresponds to a sequence comprising 24 nucleotides from a highly conserved region of the 3'-UTR of the HCV genome. The fusion gene generated in this way were cloned into the commercially available expression plasmid pcDNA3.1 (+) (Invitrogen, Life Technologies, Technologiepark Karlsruhe, Emmy-Noether-Str. 10, D-76131 Karlsruhe, Germany, catalogue number V790-20) which contains a gene for resistance to the antibiotic neomycin. The resulting plasmid is referred to as "p3." The HCV sequence shown in SEQ ID NO:7 is in p3 not part of the open reading frame of the sequence coding for β-galactosidase, so that although the HCV sequence is transcribed as part of an mRNA coding for β-galactosidase it is not expressed as part of a fusion protein. Thus, the mRNA sequence corresponding to the 24 nucleotides is identical to the corresponding sequence in the HCV genome. The plasmid p3 has the following relevant sequence segment as shown in SEQ ID NO:8 of the sequence listing:

GTC ACC TTG TCG TCA CGG CTA GCT GTG AAA GGT CCA GTC ACC ATG TCG TTT
ACT TTG

M S F T L

The N-terminal amino acid sequence of the fusion protein HCV-β-galactosidase is shown underneath the DNA sequence. The HCV sequence is depicted in italics. The start of the β-Gal gene (including 6 nucleotides of the Kozak sequence in front of the ATG codon) is underlined.

All experiments to investigate the post-transcriptional inhibition of gene expression by RNA interference were carried out with cells of the cell line β-Gal+HuH-7 which is derived from HuH-7 cells (JCRB0403, Japanese Collection of Research Bioresources Cell Bank, National Institute of Health Sciences, Kamiyoga 1-18-1, Setagaya-ku, Tokyo 158, Japan) HuH-7 is a human hepatoma cell line. The HuH-7 cells were transfected with p3. The transfected HuH-7 cells exhibit resistance to the neomycin analog G418, so that the only HuH-7 cells selected in the presence of G418 are those which have taken up the plasmid genome, and thus the reporter gene, stably in their genome. These cells are referred to herein as β-Gal+HuH-7.

The following media and solutions were used for the cell culture and the experiments:
- Dulbecco's MEN (BIOCHROM AG, Leonorenstr. 2-6, D-12247 Berlin, Germany) with 10% (v/v) fetal calf serum (FCS) and 350 µg/ml L-glutamine, referred to herein as "medium",
- Dulbecco's MEM without FCS, referred to herein as "serum-free medium" (SFM) and
- phosphate-buffered saline consisting of 137 mM NaCl, 2.7 mM KC1, 4.3 mM Na2HPO4•7 H20 and 1.4 mM KH2PO4, pH 7.3, sterilized by filtration, referred to herein as "PBS".

The cells were cultured in 10 ml of medium at 37°C with a 5% CO₂ atmosphere in 75 cm² of cell culture bottles (Coming B.V, Life Sciences, Koolhovenlaan 12, 1119 NE Schiphol-Rijk, The Netherlands).

### Transfection experiments:

Transfection experiments were carried out with β-Gal+Huh-7 cells, human cervical carcinoma cells (HELA S3 cell line, purchased from the DSMZ - Deutsche Sammlung von Mikroorganismen und Zellkulturen GmbH, Mascheroder Weg 1b, 38124 Braunschweig, Germany, order number ACC 161), human urinary bladder carcinoma cells (T-24 cell line, purchased from the DSMZ - Deutsche Sammlung von Mikroorganismen und Zellkulturen GmbH, order number ACC 376) and cells of a cell line cultured from a human pancreatic carcinoma. A bioluminescence assay was used to determine the activity of the reporter gene β-galactosidase in the β-Gal+Huh-7 cells, which enabled quantification of the inhibition efficiency of the dsRNA.

Transfections took place 24 hours after seeding of the cells in 96-well plates (cell count on the day of seeding: 2 x 10⁴ per well) or 12-well plates (cell count on the day of seeding: 1-2 x 10⁵ per well). The transfection of the cells took place either using FuGENE 6 tranfection reagent (Roche Diagnostics GmbH) or without a transfection aid.

### Transfection of β-Gal+Huh-7 cells with FuGENE 6:

Transfections were carried out in 96-well plates (quintuplicate determination) with a dsRNA concentration of 50 nM per well. The transfection mixtures which were added to the cells by pipette had a volume of 100 µl/5 wells. The transfection mixtures consisted of an amount of dsRNA corresponding to the concentration to be tested, 3 µl of FuGENE 6 per 1 µg or DNA and SFM, with which they were made up to 100 µl. The transfection was carried out by initially mixing the dsRNA in Eppendorf reaction vessels with SFN and then adding FuGENE 6. This mixture was incubated at room temperature for 20 minutes and then pipetted into the wells, and the cells were incubated at 37°C with a 5% CO₂ atmosphere for 48 hours.

### Transfection of β-Ga1+Huh-7, cervical carcinoma, urinary bladder carcinoma and pancreatic carcinoma cells without transfection aid:

Transfections were carried out in triplicate mixtures in 12-well plates. A concentration of 100 nM dsRNA was used per well. For a transfection mixture, 6 µl of a 20 µM dsRNA were mixed with 1 194 µl of SFM. The medium was then removed from the cells and replaced by 400 µl of the transfection mixture per well. After incubation at 37°C and 5% CO₂ for 2½ hours, the transfection mixture was removed by pipette and replaced by 1 ml of medium, and the cells were incubated for a further 24 hours.

### Cell lysis and bioluminescence assay:

The cell lysis was carried out 24 or 48 hours after the transfection in the 96- or 12-well plates. For this purpose, the medium was completely removed and the cells were washed twice with 100 µl or 500 µl of PBS each time. They were then covered at room temperature with 50 µl or 250 µl of lysis solution (0.5 mM 1,4-dithiothreitol (DTT) and protease inhibitor (Complete Protease Inhibitor Cocktail Tablets 50x, Roche Diagnostics GmbH, order No. 1 697 498) in the concentration recommended by the manufacturer in lysis solution (Applied Biosystems, 850 Lincoln Centre Drive, Foster City, CA 94404 USA, order No. ABX070LM) for 10 minutes and finally resuspended thoroughly with a pipette and transferred into Eppendorf reaction vessels on ice. This was followed by vortexing for 10-15 seconds and centrifugation at 16 100 g for 2 minutes. The supernatant was either used immediately for the assay or stored at -80°C.

The β-galactosidase activity was measured in 5 ml hemolysis tubes (Sarstedt AG & Co., Postfach 1220, D-51582 Nümbrecht, Germany, order No. 55.476) in a Sirius luminometer (Berthold Detection Systems GmbH, Bleichstr. 56-68, D-75173 Pforzheim, Germany). To measure the β-galactosidase activity, 5 µl of cell lysate were mixed with 30 µl of β-Gal assay buffer (100 mM Na phosphate, pH 8.0, 10 mM MgCl₂, 12.5 µg/ml Galacton (Applied Biosystems, order No. GC020)) in a hemolysis tube and incubated at room temperature for 1 hour. Then 100 µl of β-Gal stop mix (200 mM NaOH, 0.06% Emerald Enhancer II (Applied Biosystems, order No. LAY250)) were added and, after brief mixing, the luminescence was immediately measured in the luminometer.

### dsRNA isolation from cells:

24 hours after the transfection in a 12-well plate, the total RNA and the siRNA were isolated from the cell lysates using the RNeasy mini kit (QIAGEN GmbH, Max-Volmer-Straße 4, 40724 Hilden, Germany). The isolation was carried out as described in the protocol "Isolation of total RNA from animal cells" from QIAGEN, with the following modification: after the first centrifugation step, the column flow-through, which contained the short dsRNA, was stored at - 80°C until further use.

### Detection of siRNA by Northern blotting:

Since it was not possible to measure the concentration of dsRNA directly in the flow-through, it was determined in relation to the total RNA concentration. In each case identical amounts of dsRNA from the flow-throughs of the individual samples were employed. Flow-through volumes corresponding to identical dsRNA amounts were made up to identical volumes with RLT buffer (from the RNeasy mini kit)/70% ethanol 1:1, and 5 µl of E. coli tRNA (Roche Diagnostics GmbH, order No. 109541) (10 µg/µl) were added. The siRNA was precipitated by adding 1/10 volume of 3 M sodium acetate (NaOAc), pH 5.4 and 3 volumes of absolute ethanol (EtOH) at -20°C for 16 hours. The dsRNA was pelleted by centrifugation at 16 100 g for 10 minutes, and the pellets were washed with 700 µl of 70% EtOH and again centrifuged at the same g number for 5 minutes. After removal of the supernatant, the resulting dsRNA samples were resuspended in 10 µl of stop buffer (95% (v/v) formamide, 0.1% (w/v) xylene cyanole, 0.1% (w/v) bromophenol blue and 10 mM ethylenediaminetetraacetate disodium salt), denatured by incubation at 95°C for 5 minutes, placed on ice and loaded onto an 18% denaturing sequencing gel (10 x 10 x 0.8 cm, 14.5 ml of sequencing gel concentrate, 2 ml of buffer concentrate, 3.5 ml of sequencing gel diluent, each from the Rotiphorese A431.1 DNA sequencing system (Carl Roth GmbH & Co., Schoemperlenstr. 1-5, D-76185 Karlsruhe) 20 µl of *N,N,N*',*N'*,-tetramethylethylenediamine (TEMED) (Carl Roth GmbH & Co., order No. 2367.3) and 60 µl of 10% ammonium peroxodisulfate (Carl Roth GmbH & Co., order No. 9592.3). The gel was run at 150 V with 1x Tris-boric acid-EDTA (TBE) (10.8 g of Tris, 5.5 g of boric acid, 4 ml of 0.5 M EDTA, pH 8.0) as running buffer for 2-3 hours. The RNA were then transferred onto a Hybond N⁺ membrane (Amersham Biosciences Europe GmbH, order No. RPN203B) by electrophoresis (2 h at 100 mA) by the *semi-dry* method in a Hoefer semi-dry transfer unit (Amersham Biosciences Europe GmbH, Munziger Str. 9, D-79111 Freiburg, Germany, order No. 80-621-86). The membrane was then dried at room temperature for 16 hours and subsequently baked at 80°C for 3 hours.

### Preparation of the radiolabeled probes:

The probes employed for detecting the siRNA were the single-stranded RNAs Ga13s (SEQ ID NO:3) and HCV11s (SEQ ID NO:2) which were radiolabeled for this purpose at the 5' ends using T4 polynucleotide kinase (PNK). For this, 3 µl of RNA (20 µM) were mixed with 5 µl of γ-[³²P]-ATP (10 µCi/µl), 35 µl of H₂O, 5 µl of PNK 10x buffer and 1 µl of PNK (10 U/µl) (PNK 10x buffer and PNK from New England Biolabs, Bruningstr. 50, Geb. G810, D-65926 Frankfurt am Main, Germany, order No. M0201 S) and incubated at 37°C for 1 hour. The reaction mixture was then made up to 100 µl with H₂O, and the probes were purified using a MicroSpin^{™} 25 column (Amersham Biosciences Europe GmbH). This was done by loading the reaction mixture onto the columns, which had been briefly vortexed and centrifuged dry at 0.7 g for 1 minute, and the probe was eluted by centrifugation again at 0.7 g for 1 minute.

### Hybridization:

The ExpressHyb^{™} hybridization solution (BD Biosciences Clontech, Tullastr. 4, D-69126 Heidelberg, Germany) was used for the hybridization reaction. Firstly, the membrane was briefly moistened with the siRNA in H₂O and prehybridized with 5 ml of ExpressHyb hybridization solution in a shaker at 37°C for 30 minutes. The ExpressHyb hybridization solution was then replaced by a mixture of 5 ml of ExpressHyb hybridization solution and 250 ng of the radiolabeled probe, and the blot was hybridized at 37°C for 1 hour. This was followed by several washing steps at room temperature, initially with washing solution 1 (0.3 M NaCl, 30 mM sodium citrate, pH 7.0, 0.05% sodium dodecyl sulfate (SDS)), which was changed three times within 45 minutes, and then for 45 minutes with washing solution 2 (15 mM NaCl, 1.5 mM sodium citrate, pH 7.0, 0.1% SDS), exchanging the solution twice. For development, the membrane was placed on filter paper, wrapped in cling film and exposed in a cassette on a storage phosphor screen (Amersham Biosciences Europe GmbH) for 16 h. This was finally read using a Storm 820 Phosphorimager (Amersham Biosciences Europe GmbH, order No. 63-0035-52).

### Results:

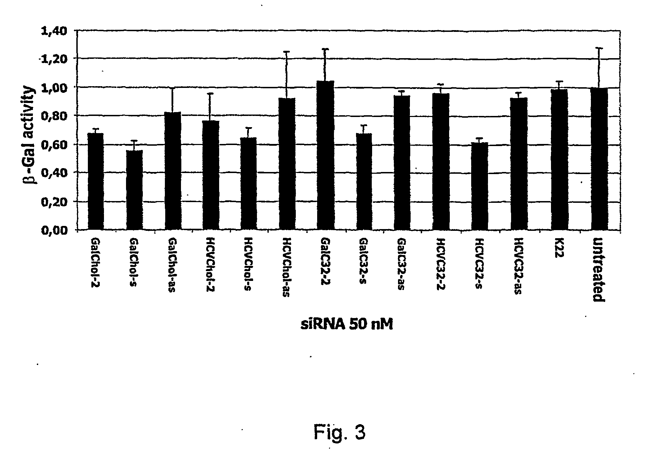

All the experiments to determine the β-galactosidase activity were carried out in quintuplicate and an average of the measured values was formed. The results for the changes in β-galactosidase activity brought about by the transfections are shown FIG. 3. The average for untreated cells was defined as 1.0, with the values for transfected cells being relative thereto. These results demonstrate that gene expression is reduced to 56% using the modified dsRNAs (see, e.g., results with GalChol-s).

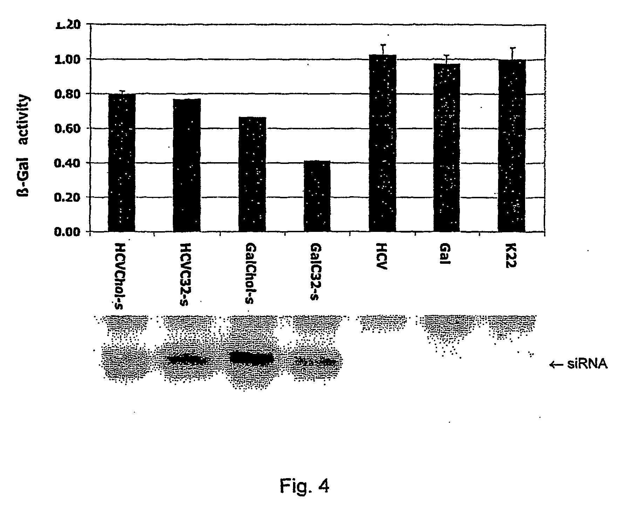

The result of the transfection experiments carried out with β-Gal+Huh-7 cells without transfection aid is shown in FIG. 4. Inhibition of β-Gal expression was determined by measuring the β-galactosidase activity. The dsRNA taken up by the cells was detected in the cell lysate by Northern blotting through hybridization with the radiolabeled single-stranded RNA probes Ga13s and HCV11s.

The upper part of FIG. 4 shows the β-Gal activity of the cells treated with the designated dsRNAs. The lower part of the figure shows the Northern blot of the dsRNA isolated from these cells. From left to right, the first four lanes of the Northern blot show the dsRNA isolates from the cells treated with the modified dsRNAs HCVChol-s, HCVC32-s, GalChol-s and Ga1C32-s. The following three lanes show dsRNA isolates from the cells treated with the unmodified dsRNAs HCV, Gal and K22.

FIG. 4 shows that dsRNAs with the Chol-s and C32-s modifications are taken up by cells even without addition of transfection aids and cause inhibition of the expression of a target gene. Unmodified dsRNAs are, by contrast, not taken up by cells without transfection aid and do not inhibit the expression of β-galactosidase.

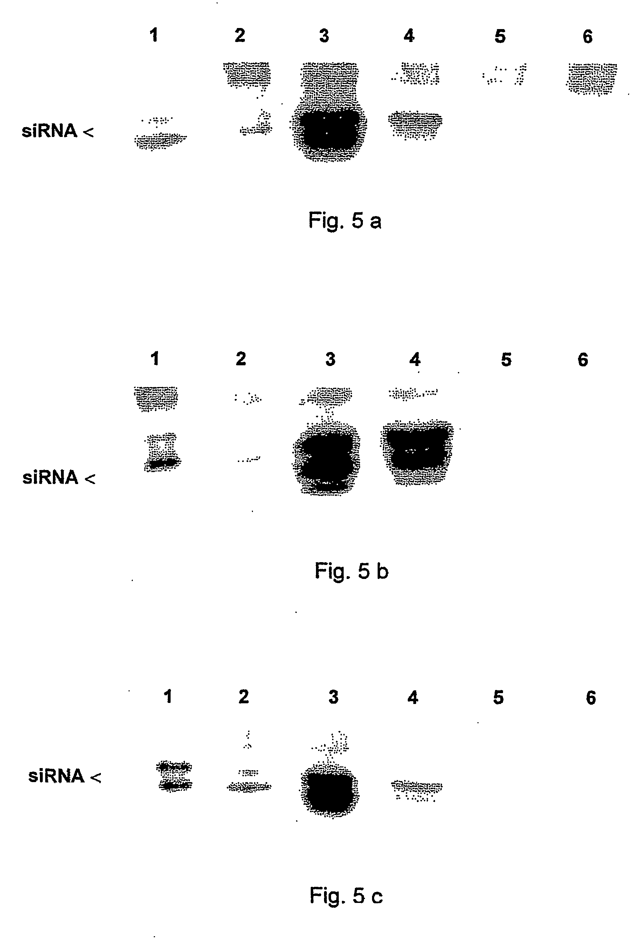

FIG. 5a, 5b and 5c shown Northern blots of dsRNA isolated from pancreatic carcinoma (FIG. 5a), cervical carcinoma (FIG. 5b) and urinary bladder carcinoma cells (FIG. 5c). The respective lanes of the Northern blots show, from left to right, dsRNA isolates from cells treated with the following dsRNAs without transfection aid: HCVChol-s (lane 1), HCVC32-s (lane 2), GalChol-s (lane 3), GalC32-s (lane 4), HCV (lane 5) and Gal (lane 6). FIG. 5a, 5b and 5c show that the dsRNA of the invention is also taken up by cells other than liver cells without transfection aid.

### Example 5: Treatment of a HCV infected Patient with dsRNA

In this Example, HCV specific double stranded RNA (dsRNA) is injected into HCV infected patients and shown to specifically inhibit HCV gene expression.

### dsRNA Administration and Dosage

The present example provides for pharmaceutical compositions for the treatment of human HCV infected patients comprising a therapeutically effective amount of a HCV specific dsRNA as disclosed herein, in combination with a pharmaceutically acceptable carrier or excipient. DsRNAs useful according to the invention may be formulated for oral or parenteral administration. The pharmaceutical compositions may be administered in any effective, convenient manner including, for instance, administration by topical, oral, anal, vaginal, intravenous, intraperitoneal, intramuscular, subcutaneous, intranasal or intradermal routes, among others. One of skill in the art can readily prepare dsRNAs for injection using such carriers that include, but are not limited to, saline, buffered saline, dextrose, water, glycerol, ethanol, and combinations thereof. Additional examples of suitable carriers are found in standard pharmaceutical texts, e.g. "Remington's Pharmaceutical Sciences", 16th edition, Mack Publishing Company, Easton, Pa., 1980.

### Example 6: HCV-specific dsRNA expression vectors

HCV-specific dsRNA molecules that interact with HCV target RNA molecules and modulate HCV gene expression activity are expressed from transcription units inserted into DNA or RNA vectors (see, for example, Couture, et al, TIG. (1996) 12:510-515, Skillern et A, International PCT Publication No. WO 00/22113, Conrad, International PCT Publication No. WO 00/22114, and Conrad, US Pat. No. 6,054,299). These transgenes can be introduced as a linear construct, a circular plasmid, or a viral vector, which can be incorporated and inherited as a transgene integrated into the host genome. The transgene can also be constructed to permit it to be inherited as an extrachromosomal plasmid (Gassmann, et al., Proc. Natl. Acad. Sci. USA (1995) 92:1292).

The individual strands of a dsRNA can be transcribed by promoters on two separate expression vectors and cotransfected into a target cell. Alternatively, each individual strand of the dsRNA can be transcribed by promoters, both of which are located on the same expression plasmid. In a preferred embodiment, the dsRNA is expressed as an inverted repeat joined by a linker polynucleotide sequence such that the dsRNA has a stem and loop structure.

The recombinant dsRNA expression vectors are preferably DNA plasmids or viral vectors. dsRNA expressing viral vectors can be constructed based on, but not limited to, adeno-associated virus (for a review, see Muzyczka, et al., Curr. Topics Micro. Immunol. (1992) 158:97-129), adenovirus (see, for example, Berkner et al., BioTechniques (1998) 6:616, Rosenfeld et al., Science (1991) 252:431-434, and Rosenfeld, et al., Cell (1992) 68:143-155), or alphavirus, as well as others known in the art. Retroviruses have been used to introduce a variety of genes into many different cell types, including epithelial cells, in vitro and/or in vivo (see, for example Eglitis, et al., Science (1985) 230:1395-1398; Danos and Mulligan, Proc. NatI. Acad. Sci. USA (1988) 85:6460-6464; Wilson, et al., Proc. NatI. Acad. Sci. USA (1988) 85:3014-3018; Armentano, et al., Proc. Natl. Acad. Sci. USA (1990) 87:61416145; Huber et al., 1991, Proc. NatI. Acad. Sci. USA 88:8039-8043; Ferry et al., 1991, Proc. NatI. Acad. Sci. USA 88:8377-8381; Chowdhury et al., 1991, Science 254:1802-1805; van Beusechem. et al., 1992, Proc. Nad. Acad. Sci. USA 89:7640-19 ; Kay et al., 1992, Human Gene Therapy 3:641-647; Dai et al., 1992, Proc. Natl.Acad. Sci. USA 89:10892-10895; Hwu et al., 1993, J. Immunol. 150:4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573). Recombinant retroviral vectors capable of transducing and expressing genes inserted into the genome of a cell can be produced by transfecting the recombinant retroviral genome into suitable packaging cell lines such as PA317 and Psi-CRIP (Comette et al., 1991, Human Gene Therapy 2:5-10; Cone et al., 1984, Proc. Natl. Acad. Sci. USA 81:6349). Recombinant adenoviral vectors can be used to infect a wide variety of cells and tissues in susceptible hosts (e.g., rat, hamster, dog, and chimpanzee) (Hsu et al., 1992, J. Infectious Disease, 166:769), and also have the advantage of not requiring mitotically active cells for infection.

The promoter driving dsRNA expression in either a DNA plasmid or viral vector of the invention may be a eukaryotic RNA polymerase I (e.g. ribosomal RNA promoter), RNA polymerase II (e.g. CMV early promoter or actin promoter or U1 snRNA promoter) or preferably RNA polymerase III promoter (e.g. U6 snRNA or 7SK RNA promoter) or a prokaryotic promoter, for example the T7 promoter, provided the expression plasmid also encodes T7 RNA polymerase required for transcription from a T7 promoter. The promoter can also direct transgene expression to the liver e.g albumin regulatory sequence (Pinkert et al., 1987, Genes Dev. 1:268276).

In addition, expression of the transgene can be precisely regulated, for example, by using an inducible regulatory sequence and expression systems such as a regulatory sequence that is sensitive to certain physiological regulators, e.g., circulating glucose levels, or hormones (Docherty et al., 1994, FASEB J. 8:20-24). Such inducible expression systems, suitable for the control of transgene expression in cells or in mammals include regulation by ecdysone, by estrogen, progesterone, tetracycline, chemical inducers of dimerization, and isopropyl-beta-D1 - thiogalactopyranoside (EPTG). A person skilled in the art would be able to choose the appropriate regulatory/promoter sequence based on the intended use of the dsRNA transgene.

Preferably, recombinant vectors capable of expressing dsRNA molecules are delivered as described below, and persist in target cells. Alternatively, viral vectors can be used that provide for transient expression of dsRNA molecules. Such vectors can be repeatedly administered as necessary. Once expressed, the dsRNAs bind to target RNA and modulate its function or expression. Delivery of dsRNA expressing vectors can be systemic, such as by intravenous or intramuscular administration, by administration to target cells ex-planted from the patient followed by reintroduction into the patient, or by any other means that allows for introduction into a desired target cell.

DsRNA expression DNA plasmids are typically transfected into target cells as a complex with cationic lipid carriers (e.g. Oligofectamine) or non-cationic lipid-based carriers (e.g. Transit-TKO^{™}). Multiple lipid transfections for dsRNA-mediated knockdowns targeting different regions of a single target gene or multiple target genes over a period of a week or more are also contemplated by the present invention. Successful introduction of the vectors of the invention into host cells can be monitored using various known methods. For example, transient transfection. can be signaled with a reporter, such as a fluorescent marker, such as Green Fluorescent Protein (GFP). Stable transfection. of ex vivo cells can be ensured using markers that provide the transfected cell with resistance to specific environmental factors (e.g., antibiotics and drugs), such as hygromycin B resistance.

The nucleic acid molecules of the invention described above can also be generally inserted into vectors and used as gene therapy vectors for human patients infected with HCV. Gene therapy vectors can be delivered to a subject by, for example, intravenous injection, local administration (see U.S. Patent 5,328,470) or by stereotactic injection (see e.g., Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

### Example 7: Synthesis of a dsRNA comprising a lipophilic group covalently attached to a 5'-end of the sense strand and demonstration of its superior efficacy and selectivity

### RNA Synthesis:

RNA is synthesized on an Expedite 8909 Synthesizer (Applied Biosystems, Applera Deutschland GmbH, Frankfurter Str. 129b, 64293 Darmstadt, Germany) at 1 µmole scale employing CPG solid support and Expedite RNA phosphoramidites (both from Proligo Biochemie GmbH, Georg-Hyken-Str.14, Hamburg, Germany). Ancillary reagents are obtained from Mallinckrodt Baker (Im Leuschnerpark 4 , 64347 Griesheim, Germany).

For 5'-derivatization the DMT off-synthesized RNA oligonucleotide still bound to the solid support is treated with a 60 µL mixture of Pyridine/Pivaloyl chloride (5/1 v/v) in Dichloromethane (500 µL) to esterify the unprotected primary alcohol. After four hours the solid support is washed with Dichloromethane (5 mL) and Acetone (10 mL) and dried by passing Argon through the synthesis column. The chemicals for these off-synthesizer manipulations are purchased from Fluka (Fluka Chemie GmbH, Industriestrasse 25, 9471 Buchs, Switzerland).

Cleavage of the modified RNA from the solid support and base deprotection is accomplished with methylamine in ethanol (Fluka Chemie GmbH, Industriestrasse 25, 9471 Buchs, Switzerland). 2'-Desilylation is carried out according to established procedures. (Wincott, F. et al., Nucleic Acids Res. (1995) 23:2677-2684). Crude oligoribonucleotides are purified by anion exchange HPLC using a 22x250 mm DNAPac PA 100 column (Dionex GmbH, Am Wörtzgarten 10, 65510 Idstein). The modified oligoribonucleotide has a longer retention time compared to the unmodified parent compound. All compounds are characterized by LC/ESI-MS (LC: Ettan Micro, Amersham Biosciences Europe GmbH, Munzinger Strasse 9, 79111 Freiburg, Germany, ESI-MS: LCQ, Deca XP, Thermo Finnigan, Im Steingrund 4-6, 63303 Dreieich, Germany).

Yields and concentrations are determined by UV absorption of a solution of the respective RNA at a wavelength of 260 nm using a spectral photometer (DU 640B, Beckman Coulter GmbH, 85702 Unterschleißheim, Germany). Double stranded RNA is generated by mixing an equimolar solution of complementary strands in annealing buffer (20 mM sodium phosphate, pH 6.8; 100 mM sodium chloride), heating in a water bath at 85 - 90 °C for 3 minutes and cooling to room temperature over a period of 3 - 4 hours. Until use the RNA is kept at -20 °C.

The target sequences in the human BCL2 mRNA are the same as in Wacheck et al. (Oligonucleotides (2003), 13:394-400) as well as the sequence of the control firefly luciferase.

### Evaluation of specificity of siRNAs

siRNAs are evaluated for specificity in HeLa cells (ATCC Number CCL-2). The cells are seeded 24 hours before treatment with siRNAs to allow adherent cell growth. Cultures are incubated for four hours with siRNAs precomplexed in opti-MEM medium with OligofectAmine (both from Invitrogen, Carlsbad, California, USA) according to the manufacturer's protocol. After incubation, the incubation medium is exchanged by complete medium and cells are cultivated under standard conditions.

Evaluation of specificity of siRNAs is performed by analyzing aliquots with the "chip" gene expression analysis technology as described, for example, in Jackson et al., Nature Biotechn. (2003) 21:635 - 638. Additional information on this technology may be obtained from, for example, "The chipping forecast", Nature Genetics, Supplement, Vol. 21, Jan. 1999 and "The chipping forecast II", Nature Genetics, Supplement, Vol. 32, Dec. 2002.

With the exclusive introduction of the 5'-Pivaloyl ester on the sense strand of the specific anti-BCL2 siRNA a significant decrease in off-target effects can be observed as analyzed using chips relative to the siRNA which does not harbour this ester.

Several groups of genes besides the target gene BCL2 show deregulation by siRNA targeted at BCL2 bearing no 5'-derivatization on its sense strand. Two groups of genes are down-regulated at a time scale similar to BCL2, one group partially complementary to the complementary strand of the siRNA, the other to the sense strand. Other groups having no sequence complementarity to either strand are deregulated with considerable time lag as compared to the former two groups and BCL2, indicating these effects to be secondary.

In contrast, treatment of cells with the siRNA comprising the 5'-derivatized sense strand results in significantly reduced down-regulation of those genes with partial sequence complementarity to the sense strand, and less genes showing secondary deregulation, indicating higher selectivity. Furthermore, the down-regulation of BCL2 proves more effective using this derivatized siRNA.

We speculate that this observation can be attributed to the missing phosphodiester or missing 5'-OH which is phosphorylated by a kinase activity inside cells as shown previously (Nykänen et al., Cell (2001) 107:309-321, Schwarz, D.S., et al., Mol. Cell (2002) 10:537-548).

## Claims

1. A double-stranded ribonucleic acid (dsRNA), comprising a complementary RNA strand, a sense RNA strand and only one lipophilic group having a logK_{ow} exceeding 1, wherein the complementary RNA strand has a nucleotide sequence which is complementary to a target RNA, and wherein the target RNA is an mRNA transcript of a target gene or of a (+) strand RNA virus, wherein the lipophilic group is covalently attached to a 5'-end of the complementary RNA strand and the linkage between the lipophilic group and the 5'-end of the complementary RNA strand comprises a phosphodiester group or wherein the lipophilic group is covalently attached to a 5'-end of the sense RNA strand.

2. The dsRNA of claim 1, wherein the linkage between the lipophilic group and the 5'-end of the sense RNA strand comprises a phosphodiester group.

3. The dsRNA of claim 1, wherein the lipophilic group is covalently attached to a 5'-end of the sense RNA strand and the linkage between the lipophilic group and the 5'-end of the sense RNA strand does not comprise a phosphodiester group.

4. The dsRNA of claim 1, wherein the complementary RNA strand comprises a 3'-end and a 5'-end, and wherein the 3'-end has a nucleotide overhang of 1 to 4 nucleotides.

5. The dsRNA of claim 1, wherein the complementary RNA strand comprises a 3'-end and a 5'-end, and wherein the 3'-end has a nucleotide overhang of 1 or 2 nucleotides.

6. The dsRNA of claim 1, wherein each of the complementary RNA strand and the sense RNA strand comprises a 3'-end and a 5'-end, wherein the lipophilic group is covalently attached to the 5'-end of the sense RNA strand, and wherein the 3'-end of the complementary RNA strand comprises a nucleotide overhang of 1 to 4 nucleotides.

7. The dsRNA of claim 6, wherein the linkage between the lipophilic group and the 5'-end of the sense strand does not comprise a phosphodiester group.

8. The dsRNA of claim 1, wherein the dsRNA is between 16 and 30 nucleotides in length.

9. The dsRNA of claim 1, wherein the dsRNA is between 16 and 25 nucleotides in length.

10. The dsRNA of claim 1, wherein the dsRNA is between 20 and 25 nucleotides in length.

11. The dsRNA of claim 1, wherein the lipophilic group is selected from the group consisting of an aromatic, aliphatic or alicyclic moiety, or a combination thereof.

12. The dsRNA of claim 1, wherein the lipophilic group is a steroid or a branched aliphatic hydrocarbon, or a combination thereof.

13. The dsRNA of claim 1, wherein the lipophilic group is a sterol.

14. The dsRNA of claim 13, wherein the sterol is cholesterol or a cholesterol derivative.

15. The dsRNA of claim 1, wherein the lipophilic group is cholesteryl (6-hydroxyhexyl) carbamate or 12-hydroxydodecanoic acid bisdecylamide.

16. The dsRNA of claim 1, wherein the target gene is expressed in a cell selected from the group consisting of a hepatocyte, a pancreatic cell, a uterine cell, a cell of a cervix, and a cell of a urinary bladder.

17. The dsRNA of claim 1, wherein the target gene is expressed in a liver cell selected from the group consisting of an endothelial cell, a Kupffer cell, and a parenchymal cell.

18. The dsRNA of claim 1, wherein the (+) strand RNA virus is a Hepatitis C Virus (HCV).

19. The dsRNA of claim 1, wherein the target gene is at least a portion of a 3'-untranslated region (3'-UTR) of a Hepatitis C Virus (HCV).

20. The dsRNA of claim 1, wherein the lipophilic group has a logK_{ow} exceeding 1.5.

21. The dsRNA of claim 1, wherein the lipophilic group has a logK_{ow} exceeding 2.

22. The dsRNA of claim 1, wherein the lipophilic group has a logK_{ow} exceeding 3.

23. A pharmaceutical composition for inhibiting the expression of a target gene in a mammal, comprising:
a) a double-stranded ribonucleic acid (dsRNA), comprising a complementary RNA strand, a sense RNA strand and only one lipophilic group having a logK_{ow} exceeding 1, wherein the complementary RNA strand has a nucleotide sequence which is complementary to an mRNA transcript of the target gene or of a (+) strand RNA virus, wherein the lipophilic group is covalently attached to a 5'-end of the complementary RNA strand and the linkage between the lipophilic group and the 5'-end of the complementary RNA strand comprises a phosphodiester group or wherein the lipophilic group is covalently attached to a 5'-end of the sense RNA strand; and
b) a pharmaceutically acceptable carrier.

24. The pharmaceutical composition of claim 23, wherein the linkage between the lipophilic group and the 5'-end of the sense RNA strand comprises a phosphodiester group.

25. The pharmaceutical composition of claim 23, wherein the linkage between the lipophilic group and the 5'-end of the sense RNA strand does not comprise a phosphodiester group.

26. The pharmaceutical composition of claim 23, wherein the complementary RNA strand comprises a 3'-end and a 5'-end, and wherein the 3'-end has a nucleotide overhang of 1 to 4 nucleotides.

27. The pharmaceutical composition of claim 23, wherein each of the complementary RNA strand and the sense RNA strand comprises a 3'-end and a 5'-end, wherein the lipophilic group is covalently attached to the 5'-end of the sense RNA strand, and wherein the 3'-end of the complementary RNA strand comprises a nucleotide overhang of 1 to 4 nucleotides.

28. The pharmaceutical composition of claim 27, wherein the linkage between the lipophilic group and the 5'-end of the sense strand does not comprise a phosphodiester group.

29. The pharmaceutical composition of claim 23, wherein the dsRNA is between 16 and 30 nucleotides in length.

30. The pharmaceutical composition of claim 23, wherein the dsRNA is between 16 and 25 nucleotides in length.

31. The pharmaceutical composition of claim 23, wherein the dsRNA is between 20 and 25 nucleotides in length.

32. The pharmaceutical composition of claim 23, wherein the lipophilic group is selected from the group consisting of an aromatic, aliphatic or alicyclic moiety, or a combination thereof.

33. The pharmaceutical composition of claim 23, wherein the lipophilic group is a steroid or a branched aliphatic hydrocarbon, or a combination thereof.

34. The pharmaceutical composition of claim 23, wherein the lipophilic group is cholesteryl (6-hydroxyhexyl) carbamate or 12-hydroxydodecanoic acid bisdecylamide.

35. The pharmaceutical composition of claim 23, wherein the target gene is expressed in a cell selected from the group consisting of a hepatocyte, a pancreatic cell, a uterine cell, a cell of a cervix, and a cell of a urinary bladder.

36. The pharmaceutical composition of claim 23, wherein the target gene is a Hepatitis C Virus (HCV).

37. The pharmaceutical composition of claim 23, wherein the target gene is at least a portion of a 3'-untranslated region (3'-UTR) of a Hepatitis C Virus (HCV).

38. The pharmaceutical composition of claim 23, wherein the pharmaceutically acceptable carrier is an aqueous solution.

39. The pharmaceutical composition of claim 23, wherein the pharmaceutically acceptable carrier does not contain an agent that mediates the uptake of the dsRNA into a cell.

40. The pharmaceutical composition of claim 23, wherein the lipophilic group has a logK_{ow} exceeding 1.5.

41. The pharmaceutical composition of claim 23, wherein the lipophilic group has a logK_{ow} exceeding 2.

42. The pharmaceutical composition of claim 23, wherein the lipophilic group has a logK_{ow} exceeding 3.

43. A method for making a double-stranded ribonucleic acid (dsRNA), comprising the steps of:
a. preparing a first (complementary) RNA strand and a second (sense) RNA strand, wherein preparing the RNA strands comprises solid-phase synthesis in a 3' to 5' direction, wherein one of the RNA strands comprises a lipophilic group having a logK_{ow} exceeding 1, wherein the lipophilic group is attached to the first (complementary) or the second (sense) RNA strand, wherein the step comprises reacting a lipophilic molecule having a phosphoramidite group with a 5'-hydroxyl group of the first or the second RNA strand; and
b. mixing the first (complementary) RNA and the second (sense) RNA strands to form a dsRNA.

44. The method of claim 43, wherein the phosphoramidite group on the lipophilic molecule is formed by phosphitylation of a hydroxy group.

45. The method of claim 43, wherein the lipophilic molecule having a phosphoramidite group is formed by converting a cholesteryl chloroformate into an amide.

46. The method of claim 43, wherein the lipophilic molecule having a phosphoramidite group is formed by reacting a 12-hydroxylauric acid with a di-n-decylamine to form an amide.

47. The method of claim 43, wherein the lipophilic molecule having a phosphoramidite group is cholesteryl N-[6-(2-cyanoethoxy)-N,N-diisopropylaminophosphanyloxy]-hexyl carbamate or 12-[(2-cyanoethoxy) -N,N-diisopropylaminophosphanyloxy]dodecanoic acid bisdecylamide.

48. The method of claim 43, wherein the lipophilic group has a logK_{ow} exceeding 1.5.

49. The method of claim 43, wherein the lipophilic group has a logK_{ow} exceeding 2.

50. The method of claim 43, wherein the lipophilic group has a logK_{ow} exceeding 3.

## Patentansprüche

1. Doppelsträngige Ribonukleinsäure (dsRNA), umfassend einen komplementären RNA-Strang, einen RNA-Sinn-Strang und nur eine lipophile Gruppe, welche einen logK_{ow} größer als 1 aufweist, wobei der komplementäre RNA-Strang eine Nukleotidsequenz aufweist, welche komplementär zu einer Ziel-RNA ist und wobei die Ziel-RNA ein mRNA-Transkript eines Zielgens oder eines (+)-Strang-RNA-Virus ist, wobei die lipophile Gruppe kovalent an ein 5'-Ende des komplementären RNA-Strangs gebunden ist und die Bindung zwischen der lipophilen Gruppe und dem 5'-Ende des komplementären RNA-Strangs eine Phosphodiestergruppe umfasst oder wobei die lipophile Gruppe kovalent an ein 5'-Ende des RNA-Sinn-Strangs gebunden ist.

2. DsRNA nach Anspruch 1, wobei die Bindung zwischen der lipophilen Gruppe und dem 5'-Ende des RNA-Sinn-Strangs eine Phosphodiestergruppe umfasst.

3. DsRNA nach Anspruch 1, wobei die lipophile Gruppe kovalent an ein 5'-Ende des RNA-Sinn-Strangs gebunden ist und die Bindung zwischen der lipophilen Gruppe und dem 5'-Ende des RNA-Sinn-Strangs keine Phosphodiestergruppe umfasst.

4. DsRNA nach Anspruch 1, wobei der komplementäre RNA-Strang ein 3'-Ende und ein 5'-Ende umfasst und wobei das 3'-Ende einen Nukleotidüberhang von 1 bis 4 Nukleotiden aufweist.

5. DsRNA nach Anspruch 1, wobei der komplementäre RNA-Strang ein 3'-Ende und ein 5'-Ende umfasst und wobei das 3'-Ende einen Nukleotidüberhang von 1 oder 2 Nukleotiden aufweist.

6. DsRNA nach Anspruch 1, wobei der komplementäre RNA-Strang und der RNA-Sinn-Strang jeweils ein 3'-Ende und ein 5'-Ende umfasst, wobei die lipophile Gruppe kovalent an das 5'-Ende des RNA-Sinn-Strangs gebunden ist und wobei das 3'-Ende des komplementären RNA-Strangs einen Nukleotidüberhang von 1 bis 4 Nukleotiden aufweist.

7. DsRNA nach Anspruch 6, wobei die Bindung zwischen der lipophilen Gruppe und dem 5'-Ende des Sinn-Strangs keine Phosphodiestergruppe umfasst.

8. DsRNA nach Anspruch 1, wobei die dsRNA zwischen 16 und 30 Nukleotiden lang ist.

9. DsRNA nach Anspruch 1, wobei die dsRNA zwischen 16 und 25 Nukleotiden lang ist.

10. DsRNA nach Anspruch 1, wobei die dsRNA zwischen 20 und 25 Nukleotiden lang ist.

11. DsRNA nach Anspruch 1, wobei die lipophile Gruppe ausgewählt ist aus der Gruppe bestehend aus einem aromatischen, aliphatischen oder alizyklischen Rest oder einer Kombination daraus.

12. DsRNA nach Anspruch 1, wobei die lipophile Gruppe ein Steroid oder ein verzweigter aliphatischer Kohlenwasserstoff oder eine Kombination daraus ist.

13. DsRNA nach Anspruch 1, wobei die lipophile Gruppe ein Sterin ist.

14. DsRNA nach Anspruch 13, wobei das Sterin Cholesterin oder ein Cholesterinderivat ist.

15. DsRNA nach Anspruch 1, wobei die lipophile Gruppe Cholesteryl-(6-hydroxyhexyl)-Carbamat oder 12-Hydroxydodekansäure-Bisdecylamid ist.

16. DsRNA nach Anspruch 1, wobei das Zielgen in einer Zelle exprimiert wird, ausgewählt aus der Gruppe bestehend aus einem Hepatozyten, einer Pankreaszelle, einer Uteruszelle, einer Zelle einer Zervix und einer Zelle einer Harnblase.

17. DsRNA nach Anspruch 1, wobei das Zielgen in einer Leberzelle, ausgewählt aus der Gruppe bestehend aus einer Endothelzelle, einer Kupfferzelle und einer Parenchymzelle, exprimiert wird.

18. DsRNA nach Anspruch 1, wobei der (+)-Strang-RNA-Virus ein Hepatitis C Virus (HCV) ist.

19. DsRNA nach Anspruch 1, wobei das Zielgen zumindest ein Teil einer 3'-untranslatierten Region (3'-UTR) eines Hepatitis C Virus ist.

20. DsRNA nach Anspruch 1, wobei die lipophile Gruppe einen 1,5 übersteigenden logK_{ow} aufweist.

21. DsRNA nach Anspruch 1, wobei die lipophile Gruppe einen 2 übersteigenden logK_{ow} aufweist.

22. DsRNA nach Anspruch 1, wobei die lipophile Gruppe einen 3 übersteigenden logK_{ow} aufweist.

23. Pharmazeutische Zusammensetzung zur Hemmung der Expression eines Zielgens in einem Säuger, umfassend:
a) eine doppelsträngige Ribonukleinsäure (dsRNA) umfassend einen komplementären RNA-Strang, einen RNA-Sinn-Strang und nur eine lipophile Gruppe, welche einen 1 übersteigenden logK_{ow} aufweist, wobei der komplementäre RNA-Strang eine Nukleotidsequenz aufweist, welche komplementär ist zu einem mRNA Transkript eines Zielgens oder eines (+)-Strang-RNA-Virus, wobei die lipophile Gruppe kovalent an ein 5'-Ende des komplementären RNA-Strangs gebunden ist und die Bindung zwischen der lipophilen Gruppe und dem 5'-Ende des komplementären RNA-Strangs eine Phosphodiestergruppe umfasst oder wobei die lipophile Gruppe kovalent an ein 5'-Ende des RNA-Sinn-Strangs gebunden ist und
b) einen pharmazeutisch verträglichen Träger.

24. Pharmazeutische Zusammensetzung nach Anspruch 23, wobei die Bindung zwischen der lipophilen Gruppe und dem 5'-Ende des RNA-Sinn-Strangs eine Phosphodiestergruppe umfasst.

25. Pharmazeutische Zusammensetzung nach Anspruch 23, wobei die Bindung zwischen der lipophilen Gruppe und dem 5'-Ende des RNA-Sinn-Strangs keine Phosphodiestergruppe umfasst.

26. Pharmazeutische Zusammensetzung nach Anspruch 23, wobei der komplementäre RNA-Strang ein 3'-Ende und ein 5'-Ende umfasst und wobei das 3'-Ende einen Nukleotidüberhang von 1 bis 4 Nukleotiden aufweist.

27. Pharmazeutische Zusammensetzung nach Anspruch 23, wobei der komplementäre RNA-Strang und der RNA-Sinn-Strang jeweils ein 3'-Ende und ein 5'-Ende umfasst, wobei die lipophile Gruppe kovalent an das 5'-Ende des RNA-Sinn-Strangs gebunden ist und wobei das 3'-Ende des komplementären RNA-Strangs einen Nukleotidüberhang von 1 bis 4 Nukleotiden umfasst.

28. Pharmazeutische Zusammensetzung nach Anspruch 27, wobei die Bindung zwischen der lipophilen Gruppe und dem 5'-Ende des Sinn-Strangs keine Phosphodiestergruppe umfasst.

29. Pharmazeutische Zusammensetzung nach Anspruch 23, wobei die dsRNA zwischen 16 und 30 Nukleotiden lang ist.

30. Pharmazeutische Zusammensetzung nach Anspruch 23, wobei die dsRNA zwischen 16 und 25 Nukleotiden lang ist.

31. Pharmazeutische Zusammensetzung nach Anspruch 23, wobei die dsRNA zwischen 20 und 25 Nukleotiden lang ist.

32. Pharmazeutische Zusammensetzung nach Anspruch 23, wobei die lipophile Gruppe ausgewählt ist aus der Gruppe bestehend aus einem aromatischen, aliphatischen oder alizyklischen Rest oder einer Kombination daraus.

33. Pharmazeutische Zusammensetzung nach Anspruch 23, wobei die lipophile Gruppe ein Steroid oder ein verzweigter aliphatischer Kohlenwasserstoff oder eine Kombination daraus ist.

34. Pharmazeutische Zusammensetzung nach Anspruch 23, wobei die lipophile Gruppe Cholesteryl-(6-hydroxyhexyl)-Carbamat oder 12-Hydroxydodekansäure-Bisdecylamid ist.

35. Pharmazeutische Zusammensetzung nach Anspruch 23, wobei das Zielgen in einer Zelle exprimiert wird, ausgewählt aus der Gruppe bestehend aus einem Hepatozyten, einer Pankreaszelle, einer Uteruszelle, einer Zelle einer Zervix und einer Zelle einer Harnblase.

36. Pharmazeutische Zusammensetzung nach Anspruch 23, wobei das Zielgen ein Hepatitis C Virus (HCV) ist.

37. Pharmazeutische Zusammensetzung nach Anspruch 23, wobei das Zielgen zumindest ein Teil einer 3'-untranslatierten Region (3'-UTR) eines Hepatitis C Virus (HCV) ist.

38. Pharmazeutische Zusammensetzung nach Anspruch 23, wobei der pharmazeutisch verträgliche Träger eine wässrige Lösung ist.

39. Pharmazeutische Zusammensetzung nach Anspruch 23, wobei der pharmazeutisch verträgliche Träger kein Agens enthält, welches die Aufnahme der dsRNA in eine Zelle vermittelt.

40. Pharmazeutische Zusammensetzung nach Anspruch 23, wobei die lipophile Gruppe einen 1,5 übersteigenden logK_{ow} aufweist.

41. Pharmazeutische Zusammensetzung nach Anspruch 23, wobei die lipophile Gruppe einen 2 übersteigenden logK_{ow} aufweist.

42. Pharmazeutische Zusammensetzung nach Anspruch 23, wobei die lipophile Gruppe einen 3 übersteigenden logK_{ow} aufweist.

43. Verfahren zur Herstellung einer doppelsträngigen Ribonukleinsäure (dsRNA) umfassend die Schritte:
a. Herstellen eines ersten (komplementären) RNA-Strangs und eines zweiten RNA-(Sinn)-Strangs, wobei das Herstellen der RNA-Stränge eine Festphasensynthese in eine 3'- nach 5'-Richtung umfasst, wobei einer der RNA-Stränge eine lipophile Gruppe umfasst, welche einen 1 übersteigenden logK_{ow} aufweist, wobei die lipophile Gruppe an den ersten (komplementären) oder den zweiten RNA-(Sinn)-Strang gebunden wird, wobei es der Schritt umfasst, dass ein eine Phosphoramiditgruppe aufweisendes lipophiles Molekül mit einer 5'-Hydroxylgruppe des ersten oder zweiten RNA-Strangs reagiert und
b. Mischen des ersten (komplementären) RNA- und des zweiten RNA-(Sinn)-Strangs zur Bildung einer dsRNA.

44. Verfahren nach Anspruch 43, wobei die Phosphoramiditgruppe an dem lipophilen Molekül durch Phosphorylierung einer Hydroxygruppe gebildet wird.

45. Verfahren nach Anspruch 43, wobei das eine Phosphoramiditgruppe aufweisende lipophile Molekül durch Konvertieren eines Cholesteryl-Chloroformats in ein Amid gebildet wird.

46. Verfahren nach Anspruch 43, wobei das eine Phosphoramiditgruppe aufweisende lipophile Molekül durch Reagieren einer 12-Hydroxylaurylsäure mit einem Di-N-Decylamin zur Bildung eines Amids gebildet wird.

47. Verfahren nach Anspruch 43, wobei das die Phosphoramiditgruppe aufweisende lipophile Molekül Cholesteryl-N-[6-(2-Cyanoethoxy)-N,N-Diisopropylaminophosphanyloxy]-Hexyl-Carbamat oder 12-[(2-Cyanoethoxy)-N,N-Diisopropylaminophosphanyloxy]Dodekansäure-Bisdecylamid ist.

48. Verfahren nach Anspruch 43, wobei die lipophile Gruppe einen 1,5 übersteigenden logK_{ow} aufweist.

49. Verfahren nach Anspruch 43, wobei die lipophile Gruppe einen 2 übersteigenden logK_{ow} aufweist.

50. Verfahren nach Anspruch 43, wobei die lipophile Gruppe einen 3 übersteigenden logK_{ow} aufweist.

## Revendications

1. Acide ribonucléique double brin (ARNdb), comprenant un brin d'ARN complémentaire, un brin d'ARN sens et seulement un groupe lipophile ayant un logK_{ow} supérieur à 1, dans lequel le brin d'ARN complémentaire a une séquence de nucléotides qui est complémentaire d'un ARN cible, et dans lequel l'ARN cible est un transcrit d'ARNm d'un gène cible ou d'un virus à ARN à brin (+), dans lequel le groupe lipophile est lié de manière covalente à une extrémité 5' du brin d'ARN complémentaire et la liaison entre le groupe lipophile et l'extrémité 5' du brin d'ARN complémentaire comprend un groupe phosphodiester ou dans lequel le groupe lipophile est lié de manière covalente à une extrémité 5' du brin d'ARN sens.

2. ARNdb selon la revendication 1, dans lequel la liaison entre le groupe lipophile et l'extrémité 5' du brin d'ARN sens comprend un groupe phosphodiester.

3. ARNdb selon la revendication 1, dans lequel le groupe lipophile est lié de manière covalente à une extrémité 5' du brin d'ARN sens, et la liaison entre le groupe lipophile et l'extrémité 5' du brin d'ARN sens ne comprend pas de groupe phosphodiester.

4. ARNdb selon la revendication 1, dans lequel le brin d'ARN complémentaire comprend une extrémité 3' et une extrémité 5', et dans lequel l'extrémité 3' a un débord nucléotidique de 1 à 4 nucléotides.

5. ARNdb selon la revendication 1, dans lequel le brin d'ARN complémentaire comprend une extrémité 3' et une extrémité 5', et dans lequel l'extrémité 3' a un débord nucléotidique de 1 ou 2 nucléotides.

6. ARNdb selon la revendication 1, dans lequel chacun du brin d'ARN complémentaire et du brin d'ARN sens comprend une extrémité 3' et une extrémité 5', dans lequel le groupe lipophile est lié de manière covalente à l'extrémité 5' du brin d'ARN sens, et dans lequel l'extrémité 3' du brin d'ARN complémentaire comprend un débord nucléotidique de 1 à 4 nucléotides.

7. ARNdb selon la revendication 6, dans lequel la liaison entre le groupe lipophile et l'extrémité 5' du brin sens ne comprend pas de groupe phosphodiester.

8. ARNdb selon la revendication 1, dans lequel l'ARNdb est d'une longueur comprise entre 16 et 30 nucléotides.

9. ARNdb selon la revendication 1, dans lequel l'ARNdb est d'une longueur comprise entre 16 et 25 nucléotides.

10. ARNdb selon la revendication 1, dans lequel l'ARNdb est d'une longueur comprise entre 20 et 25 nucléotides.

11. ARNdb selon la revendication 1, dans lequel le groupe lipophile est choisi dans le groupe constitué par un groupement ou partie aromatique, aliphatique ou alicyclique, ou une combinaison de ceux-ci.

12. ARNdb selon la revendication 1, dans lequel le groupe lipophile est un stéroïde ou un hydrocarbure aliphatique ramifié, ou une combinaison de ceux-ci.

13. ARNdb selon la revendication 1, dans lequel le groupe lipophile est un stérol.

14. ARNdb selon la revendication 13, dans lequel le stérol est le cholestérol ou un dérivé du cholestérol.

15. ARNdb selon la revendication 1, dans lequel le groupe lipophile est le carbamate de (6-hydroxyéthyl)cholestéryle ou le bisdécylamide de l'acide 12-hydroxydodécanoïque.

16. ARNdb selon la revendication 1, dans lequel le gène cible est exprimé dans une cellule choisie dans le groupe constitué par un hépatocyte, une cellule pancréatique, une cellule utérine, une cellule du col de l'utérus, et une cellule de la vessie urinaire.

17. ARNdb selon la revendication 1, dans lequel le gène cible est exprimé dans une cellule hépatique choisie dans le groupe constitué par une cellule endothéliale, une cellule de Kupffer, et une cellule parenchymateuse.

18. ARNdb selon la revendication 1, dans lequel le virus à ARN à brin (+) est un virus de l'hépatite C (VHC).

19. ARNdb selon la revendication 1, dans lequel le gène cible est au moins une partie d'une région non traduite en 3' (3'-UTR) d'un virus de l'hépatite C (VHC).

20. ARNdb selon la revendication 1, dans lequel le groupe lipophile a un logK_{ow} supérieur à 1,5.

21. ARNdb selon la revendication 1, dans lequel le groupe lipophile a un logK_{ow} supérieur à 2.

22. ARNdb selon la revendication 1, dans lequel le groupe lipophile a un logK_{ow} supérieur à 3.

23. Composition pharmaceutique destinée à inhiber l'expression d'un gène cible chez un mammifère, qui comprend :
a) un acide ribonucléique double brin (ARNdb) comprenant un brin d'ARN complémentaire, un brin sens et uniquement un groupe lipophile ayant un logK_{ow} supérieur à 1, dans lequel le brin d'ARN complémentaire a une séquence de nucléotides qui est complémentaire d'un transcrit d'ARNm du gène cible ou d'un virus à ARN à brin (+), dans lequel le groupe lipophile est lié de manière covalente à une extrémité 5' du brin d'ARN complémentaire et la liaison entre le groupe lipophile et l'extrémité 5' du brin d'ARN complémentaire comprend un groupe phosphodiester ou dans lequel le groupe lipophile est lié de manière covalente à une extrémité 5' du brin d'ARN sens ; et
b) un vecteur pharmaceutiquement acceptable.

24. Composition pharmaceutique selon la revendication 23, dans laquelle la liaison entre le groupe lipophile et l'extrémité 5' du brin d'ARN sens comprend un groupe phosphodiester.

25. Composition pharmaceutique selon la revendication 23, dans laquelle liaison entre le groupe lipophile et l'extrémité 5' du brin d'ARN sens ne comprend pas de groupe phosphodiester.

26. Composition pharmaceutique selon la revendication 23, dans laquelle le brin d'ARN complémentaire comprend une extrémité 3' et une extrémité 5', et dans laquelle l'extrémité 3' a un débord nucléotidique de 1 à 4 nucléotides.

27. Composition pharmaceutique selon la revendication 23, dans laquelle chacun du brin d'ARN complémentaire et du brin d'ARN sens comprend une extrémité 3' et une extrémité 5', dans laquelle le groupe lipophile est lié de manière covalente à l'extrémité 5' du brin d'ARN sens, et dans laquelle l'extrémité 3' du brin d'ARN complémentaire comprend un débord nucléotidique de 1 à 4 nucléotides.

28. Composition pharmaceutique selon la revendication 27, dans laquelle la liaison entre le groupe lipophile et l'extrémité 5' du brin sens ne comprend pas de groupe phosphodiester.

29. Composition pharmaceutique selon la revendication 23, dans laquelle l'ARNdb est d'une longueur comprise entre 16 et 30 nucléotides.

30. Composition pharmaceutique selon la revendication 23, dans laquelle l'ARNdb est d'une longueur comprise entre 16 et 25 nucléotides.

31. Composition pharmaceutique selon la revendication 23, dans laquelle l'ARNdb est d'une longueur comprise entre 20 et 25 nucléotides.

32. Composition pharmaceutique selon la revendication 23, dans laquelle le groupe lipophile est choisi dans le groupe constitué par un groupement ou une partie aromatique, aliphatique ou alicyclique, ou une combinaison de ceux-ci.

33. Composition pharmaceutique selon la revendication 23, dans laquelle le groupe lipophile est un stéroïde ou un hydrocarbure aliphatique ramifié, ou une combinaison de ceux-ci.

34. Composition pharmaceutique selon la revendication 23, dans laquelle le groupe lipophile est le carbamate de (6-hydroxyéthyl)cholestéryle ou le bisdécylamide de l'acide 12-hydroxydodécanoïque.

35. Composition pharmaceutique selon la revendication 23, dans laquelle le gène cible est exprimé dans une cellule choisie dans le groupe constitué par un hépatocyte, une cellule pancréatique, une cellule utérine, une cellule du col de l'utérus, et une cellule de la vessie urinaire.

36. Composition pharmaceutique selon la revendication 23, dans laquelle le gène cible est un virus de l'hépatite C (VHC).

37. Composition pharmaceutique selon la revendication 23, dans laquelle le gène cible est au moins une partie ou portion d'une région non traduite en 3' (3'-UTR) d'un virus de l'hépatite C (VHC).

38. Composition pharmaceutique selon la revendication 23, dans laquelle le vecteur pharmaceutiquement acceptable est une solution aqueuse.

39. Composition pharmaceutique selon la revendication 23, dans laquelle le vecteur pharmaceutiquement acceptable ne contient pas d'agent qui médie l'absorption de l'ARNdb dans une cellule.

40. Composition pharmaceutique selon la revendication 23, dans laquelle le groupe lipophile a un logK_{ow} supérieur à 1,5.

41. Composition pharmaceutique selon la revendication 23, dans laquelle le groupe lipophile a un logK_{ow} supérieur à 2.

42. Composition pharmaceutique selon la revendication 23, dans laquelle le groupe lipophile a un logK_{ow} supérieur à 3.

43. Procédé de fabrication d'un acide ribonucléique double brin (ARNdb), qui comprend les étapes consistant à :
a. préparer un premier brin (complémentaire) d'ARN et un second brin (sens) d'ARN, dans lequel la préparation des brins d'ARN comprend la synthèse en phase solide dans une direction de 3' vers 5', dans lequel un des brins d'ARN comprend un groupe lipophile ayant un logK_{ow} supérieur à 1, dans lequel le groupe lipophile est lié au premier brin (complémentaire) ou au second brin (sens) d'ARN, dans lequel l'étape comprend la réaction d'une molécule lipophile ayant un groupe phosphoramidite avec un groupe hydroxyle en 5' du premier ou du second brin d'ARN ; et
b. mélanger le premier brin (complémentaire) d'ARN et le second brin (sens) d'ARN pour former un ARNbd.

44. Procédé selon la revendication 43, dans lequel le groupe phosphoramidite sur la molécule lipophile est formé par phosphitylation d'un groupe hydroxy.

45. Procédé selon la revendication 43, dans lequel la molécule lipophile ayant un groupe phosphoramidite est formée par conversion d'un chloroformate de cholestéryle en un amide.

46. Procédé selon la revendication 43, dans lequel la molécule lipophile ayant un groupe phosphoramidite est formée par réaction d'un acide 12-hydroxylaurique avec une di-n-décylamine pour former un amide.

47. Procédé selon la revendication 43, dans lequel la molécule lipophile ayant un groupe phosphoramidite est le carbamate de N-[6-(2-cyanoéthoxy)-N,N-diisopropylaminophosphanyloxy]-hexyl cholestéryle ou le bisdécylamide de l'acide 12-[(2-cyanoéthoxy)-N,N-diisopropylamino-phosphanyloxy] dodécanoïque.

48. Procédé selon la revendication 43, dans lequel le groupe lipophile a un logK_{ow} supérieur à 1,5.

49. Procédé selon la revendication 43, dans lequel le groupe lipophile a un logK_{ow} supérieur à 2.

50. Procédé selon la revendication 43, dans lequel le groupe lipophile a un logK_{ow} supérieur à 3.


### SEQUENCE LISTING

<110> Ribopharma AG
<120> Double-stranded ribonucleic acid with improved activity
<130> 422517EH
<160> 8
<170> PatentIn version 3.1
<210> 1
   <211> 23
   <212> RNA
   <213> Hepatitis C virus
<400> 1
   ggaccuuuca cagcuagccg uga 23
<210> 2
   <211> 21
   <212> RNA
   <213> Hepatitis C virus
<400> 2
   acggcuagcu gugaaagguc c 21
<210> 3
   <211> 23
   <212> RNA
   <213> Escherichia coli
<400> 3
   cacgcucauc gauaauuuca ccg 23
<210> 4
   <211> 21
   <212> RNA
   <213> Escherichia coli
<400> 4
   gugaaauuau cgaugagcgu g 21
<210> 5
   <211> 24
   <212> RNA
   <213> Artificial sequence
<400> 5
   gaugaggauc guuucgcaug auug 24
<210> 6
   <211> 24
   <212> RNA
   <213> Artificial sequence
<400> 6
   aucaugcgaa acgauccuca uccu 24
<210> 7
   <211> 24
   <212> DNA
   <213> Hepatitis C virus
<400> 7
   gtcacggcta gctgtgaaag gtcc 24
<210> 8
   <211> 57
   <212> DNA
   <213> Artificial sequence
<400> 8
   gtcaccttgt cgtcacggct agctgtgaaa ggtccagtca ccatgtcgtt tactttg 57

### SEQUENCE LISTING

<110> Ribopharma AG
<120> Double-stranded ribonucleic acid with improved activity
<130> 422517EH
<160> 8
<170> Patent In version 3.1
<210> 1
   <211> 23
   <212> RNA
   <213> Hepatitis C virus
<400> 1
   ggaccuuuca cagcuagccg uga 23
<210> 2
   <211> 21
   <212> RNA
   <213> Hepatitis C virus
<400> 2
   acggcuagcu gugaaagguc c 21
<210> 3
   <211> 23
   <212> RNA
   <213> Escherichia coli
<400> 3
   cacgcucauc gauaauuuca ccg 23
<210> 4
   <211> 21
   <212> RNA
   <213> Escherichia coli
<400> 4
   gugaaauuau cgaugagcgu g 21
<210> 5
   <211> 24
   <212> RNA
   <213> Artificial sequence
<400> 5
gaugaggauc guuucgcaug auug 24
<210> 6
   <211> 24
   <212> RNA
   <213> Artificial sequence
<400> 6
   aucaugcgaa acgauccuca uccu 24
<210> 7
   <211> 24
   <212> DNA
   <213> Hepatitis C virus
<400> 7
   gtcacggcta gctgtgaaag gtcc 24
<210> 8
   <211> 57
   <212> DNA
   <213> Artificial sequence
<400> 8
   gtcaccttgt cgtcacggct agctgtgaaa ggtccagtca ccatgtcgtt tactttg 57